# EUROPEAN PATENT SPECIFICATION

(11) **EP 1 934 327 B1**
(45) Date of publication and mention of the grant of the patent: **09.01.2013**
(21) Application number: 06792041.3
(22) Date of filing: 13.09.2006
(51) Int. Cl.: C12N 1/20, A61K 8/99, A61Q 15/00

(54) **MICROORGANISMS INHIBITING THE FORMATION OF AXILLARY MALODOR**
DIE BILDUNG VON SCHLECHTEM ACHSELGERUCH HEMMENDE MIKROORGANISMEN
MICRO-ORGANISMES INHIBANT LA FORMATION D UNE MAUVAISE ODEUR AXILLAIRE

(30) Priority: 13.09.2005 EP 05019924; 28.11.2005 US 740225 P
(43) Date of publication of application: 25.06.2008
(73) Proprietor: BASF SE, 67056 Ludwigshafen (DE)
(72) Inventor: LANG, Christine, 10625 Berlin (DE); VEEN, Markus, 84453 Altmühldorf/Inn (DE); BUDDE, Eckhard, 50733 Köln (DE); BÖTTNER, Mewes, 10407 Berlin (DE); REINDL, Andreas, 68199 Mannheim (DE); KNÖLL, Rolf, 69514 Laudenbach (DE)
(74) Representative: Jungblut, Bernhard Jakob
(86) International application number: PCT/EP2006/008923
(87) International publication number: WO 2007/031300

(56) References cited:
- WO-A2-00/78322
- GB-A- 1 584 694
- US-A1- 2002 168 388
- US-A1- 2005 019 894
- SONG Y-L ET AL: "Rapid identification of 11 human intestinal Lactobacillus species by multiplex PCR assays using group- and species-specific primers derived from the 16S-23S rRNA intergenic spacer region and its flanking 23S rRNA" FEMS MICROBIOLOGY LETTERS, AMSTERDAM, NL, vol. 187, no. 2, 15 June 2000 (2000-06-15), pages 167-173, XP002225666 ISSN: 0378-1097
- SCHLEIFER K H ET AL: "Phylogeny of the genus Lactobacillus and related genera" SYSTEMATIC AND APPLIED MICROBIOLOGY, vol. 18, no. 4, 1995, pages 461-467, XP009074893 ISSN: 0723-2020
- NATSCH ANDREAS ET AL: "A specific bacterial aminoacylase cleaves odorant precursors secreted in the human axilla" JOURNAL OF BIOLOGICAL CHEMISTRY, AMERICAN SOCIETY OF BIOLOCHEMICAL BIOLOGISTS, BIRMINGHAM,, US, vol. 278, no. 8, 21 February 2003 (2003-02-21), pages 5718-5727, XP002233274 ISSN: 0021-9258 cited in the application

## Description

The present invention relates to the use of microorganisms selected from the group of claim 1 which are able to suppress the production of malodorous compounds by axillary bacteria for the preparation of a cosmetic or pharmaceutical composition for suppressing axillary odor.

It is generally known in the art that fresh extracted axillary sweat is odorless. According to Leyden et al. (J. Invest. Dermatol. 77 (1981), 413-416) axillary odor occurs due to bacterial degradation of sweat. It is known that the typical strong axilla odor can only be released from apocrine secretions and that the action of skin bacteria is needed to generate the odoriferous compounds from non-smelling molecules present in these secretions (Shelly et al., Arch. Dermatol. Syphilol. 68 (1953), 430-446). Only when bacteria colonizing the axilla contact the odor precursors the typically axillary sweat odor occurs. The axilla is a skin region supporting a dense bacterial population, which is dominated by the genera *Staphylococcus* and *Corynebacteria.* Most individuals carry a flora that is dominated by either one of these two genera and a strong correlation was found between a high population of *Corynebacteria* and a strong axillary odor formation (Leyden et al., 1981; Natsch et al., J. Biol. Chem. 278(8) (2003), 5718-5727). Thus, axilla secretions contain non-odoriferous precursors that are transformed by bacterial enzymes mainly present in *Corynebaderia* species.

Several odoriferous compounds in human body odor have been identified. Eearly studies characterized odoriferous steroids of the androstenone-type which were released from secreted sulfates and glucuronides in the axilla as causal agents of axillary malodor. As further important odor components thiols were identified which are produced by amino acid β-lyases that act in the axilla to cleave amino acids with the general structure COO-H-CH(NH₂)-CH₂-S-R. The resulting volatile sulfur products are thought to be responsible for the pungent smell characteristic of human axillary odor (US 5,213,781). Zeng et al. (J. Chem. Ecol. 18 (1991), 1039-1055) proposed that short, branched fatty acids act as key components of axillary malodor with 3-methyl-2-hexenoic acid (3M2H) being the major contribuent. They could further show that 3M2H is non-covalently associated with apolipoprotein D, the major protein present in axilla secretions and that 3M2H is released upon incubation of the aqueous fraction of apocrine secretions with *Corynebacteria,* indicating that a watersoluble, covalently-linked precursor must be present. Natsch et al. (2003) could demonstrate that 3M2H as well as a chemically related compound, 3-hydroxy-3-methylhexanoic acid (HMHA), are covalently linked to a glutamine residue in fresh axilla secretions. These non-odoriferous precursor, 3-methyl-2-hexenoyl-L-glutamine (3M2H-Gln) and 3-hydroxy-3-methylhexanoyl-L-glutamine (HMHA-Gln) were shown to be cleaved by an N-acyl-aminoacylase enzyme purified from a *Corynebacterium striatum* strain. This enzyme is therefore hypothesized to be the main originator of the short, branched fatty acid-type of malodorous compounds.

The first deodorants relied on strong perfumes to mask body odor. Most of current cosmetic deodorants available on the market, however, rely on an unspecific inhibition of the biological activity of axillary microorganisms, i.e. the eradication of the bacteria responsible for causing the odor. In fact, cosmetic deodorants generally contain antibacterial compounds which inhibit the growth of the skin micro flora, like, for instance, 2,4,4'-trichloro-2'hydroxy-diphenyl-ether (Triclosan). However, while this principle is effective against odor formation, it leads to a severe destruction of the natural residential microbial skin flora that protects the skin, e.g. from being colonized by potentially pathogenic microorganisms (Bisno et al., Am. J. Med. 76 (5A) (1984), 172-179). An alternative which was taken into consideration is the direct inhibition of the action of enzymes known to be involved in the generation of malodorous compounds (EP 1 258 531). However, many of these inhibitors like, e.g. phenantroline, DTT or CuSO₄ which could be demonstrated to be potent inhibitors of the 3M2H releasing enzyme N-acyl-aminoacylase are potentially noxious and therefore unsuitable for a topical application on the axillary skin. In vivo and in vitro experimental studies report that zinc chelators like EDTA, EGTA or others have severe tissue and skin irritating properties due to their property to reduce the skins barrier function (Braz, Dent. J. 16, (2005), 3-8). Also dithiols like dithiothreitol are commonly known to irritate the skin. Acyl-L-glutamates like lauroyl-L-glutamine or caramates of glutamine have been described as unsafe (see Sigma-Aldrich MSDS safety reports 43817 and 61732). Additionally all these substances have a potential to disturb the natural commensal skin microbial flora which is involved in maintaining the important skin barrier function (Bisno et al., Am. J. Med. 76 (5A) (1984), 172-179).

Thus, there is a need for means and methods allowing to suppress the release of malodorous compounds without causing severe side-effects or adversely affecting the microbial skin flora of the human axilla.

The present invention addresses this need and provides microorganisms and methods which lead to the suppression of the release of 3-methyl-2-hexenoic acid and its odorous derivatives In particular, it provides the embodiments as characterized in the claims.

Accordingly, the present invention relates to the use of a microorganism which is able to suppress the release of 3-methyl-2-hexenoic acid or its odorous derivatives by axillary bacteria, selected from the group of claim 1.

The inventors surprisingly found that an effective suppression of the release of 3-methyl-2-hexenoic acid or its odorous derivatives can be achieved by applying to the axilla the above described microorganisms or inactivated forms thereof. The inventors for the first time identified corresponding microorganisms and provided methods for their identification. These microorganisms are able to biochemically suppress the conversion of odourless precursor compounds to 3-methyl-2-hexenoic acid or odoriferous derivatives thereof. By this the generation of axillary malodour is prevented.

The term "odorous derivative of 3-methyl-2-hexenoic acid" relates to compounds which are chemically derivable from 3-methyl-2-hexenoic acid or structurally related to 3-methyl-2-hexenoic and which are odorous. Preferably, the term relates to compounds selected from the group consisting of 3-methyl-2-hexanoic acid, 3-hydroxy-3-methyl-hexanoic acid, (*E*)-3-methyl-2-hexenoic acid (*E*-3M2H) and (*Z*)-3-methyl-2-hexenoic acid (*Z*-3M2H).

The term "release of 3-methyl-2-hexenoic acid or its odorous derivatives" relates to the conversion of an odourless precursor which can normally be found in axillary secret into 3-methyl-2-hexenoic acids or an odorous derivative thereof.

In particular, the term "odourless precursor" relates to a chemical compound which is per se not odoriferous, but becomes odoriferous when it is chemically or enzymatically converted in a reaction which leads to the production of 3-methyl-2-hexenoic acids or an odorous derivative thereof. Preferably, the term "odourless precursor" relates to 3-methyl-2-hexenoyl-L-glutamine (3M2H-Gln), 3-methyl-2-hexenoyl-L-apolipoprotein D (3M2H-apoD), 3-methyl-2-hexenoyl-L-ASOP1, 3-methyl-2-hexenoyl-L-ASOP2, 3-hydroxy-3-methylhexanoyl-L-glutamine (HMHA-Gln), 3-hydroxy-3-methylhexanoyl-L-apolipoprotein D (HMHA-apoD), 3-hydroxy-3-methylhexanoyl-L-ASOP1 or 3-hydroxy-3-methylhexanoyl-L-ASOP2.

The term "odorous" or "odoriferous" means that a typical axillar sweat odor can be detected. Preferably, the term means that the detection of the typical axillar sweat odor is verified by sniffing with the nose, preferably the nose of a skilled person. More preferably, the term refers to the amount of 3-methyl-2-hexenoic acid or derivatives thereof which can be detected by GC/MS analysis. The term "odourless" means that a typical axillar sweat odor cannot be detected be detected by sniffing with the nose, preferably the nose of a skilled person. More preferably, the term means that no 3-methyl-2-hexenoic acid or derivatives thereof can be detected by GC/MS analysis. The verification by "sniffing with the nose" relates to a detection of typical axillar sweat odor carried out by one or more persons having been trained for the detection of odor with their noses. The detection may be carried out in any suitable form or by using any suitable technique known to the person skilled in the art. Preferably the detection may be carried out by a qualified panel of persons having been trained for the detection of axillary odor with their noses, more preferably it may be carried out by three persons which form a qualified panel. There are different categories of odor intensity. Preferably these categories may be defined as: 0=no odor detectable, 1=slight odor detectable, 2=odor detectable and 3=strong odor detectable. The person or persons forming the qualified panel may independently assess the odor intensity of odorous samples of microorganisms. Preferably the odor of in vitro generated samples consisting of microorganisms, able to release 3M2H from its precursor form, non odorous axillary secret and a microorganism able to suppress the release of 3M2H or corresponding control samples without microorganisms defined in the invention may be assessed. The value of odor perception of the person(s) belonging to the qualified panel may be calculated by any means known to the person skilled in the art. Preferably, the mean value of odor perception of all person(s) belonging to the qualified panel may be calculated. Based on these data the intensity of odor may subsequently be quantified by any means known to the person skilled in the art.

The term "odorous derivatives" or "derivatives of 3-methyl-2-hexenoic acid" relates to derivatives of 3-methyl-2-hexenoic acid which generate a typical axillar sweat odor as can be verified by sniffing with the nose, preferably the nose of the skilled person. Preferably, the term "derivatives of 3-methyl-2-hexenoic acid" relates to compounds which are chemically derivable from 3-methyl-2-hexenoic acid or structurally related to 3-methyl-2-hexenoic and which are odorous. More preferably, the term relates to compounds selected from the group consisting of 3-methyl-2-hexanoic acid, 3-hydroxy-3-methyl-hexanoic acid, (*E*)-3-methyl-2-hexenoic acid (*E*-3M2H) and (*Z*)-3-methyl-2-hexenoic acid (*Z*-3M2H).

The term "skin" refers to the body's outer covering, as known to the person skilled in the art. Preferably the term relates to three layers: epidermis, dermis, and subcutaneous fatty tissue. The epidermis is the outermost layer of the skin. It typically forms the waterproof, protective wrap over the body's surface and is made up of stratified squamous epithelium with an underlying basal lamina. It usually contains no blood vessels, and is nourished by diffusion from the dermis. The main type of cells which make up the epidermis are keratinocytes, with melanocytes and Langerhans cells also present. The epidermis is divided into several layers where cells are formed through mitosis at the innermost layers. They move up the strata changing shape and composition as they differentiate and become filled with keratin. They eventually reach the top layer called stratum corneum and become sloughed off, or desquamated. The outermost layer of the epidermis consists of 25 to 30 layers of dead cells. Conventionally, the epidermis is divided into 5 sublayers or strata (from superficial to deep): the stratum corneum, the stratum lucidum, the stratum granulosum, the stratum spinosum and the stratum germinativum or stratum basale. Typically, the interface between the epidermis and dermis is irregular and consists of a succession of papillae, or fingerlike projections, which are smallest where the skin is thin and longest in the skin of the palms and soles. Typically, the papillae of the palms and soles are associated with elevations of the epidermis, which produce ridges. Subcutaneous fatty tissue is the deepest layer of the skin. A characteristic of this layer is that it is composed of connective tissue, blood vessels, and fat cells. Typically, this layer binds the skin to underlying structures, insulates the body from cold, and stores energy in the form of fat. In general the skin forms a protective barrier against the action of physical, chemical, and bacterial agents on the deeper tissues. This means that tissues belonging , e.g. to the oral cavity or the vaginal region or mucous membranes do not belong to the skin. In a preferred embodiment the term "skin" relates to the outermost layer of the body's covering, i.e. the epidermis. In a more preferred embodiment the term "skin" relates to the stratum corneum of the epidermis. In an even more preferred embodiment the term "skin" relates to the outermost 25 to 30 layers of dead cells of the epidermis. In the most preferred embodiment the term "skin" relates to the outermost 10 layers of dead cell of the epidermis.

In another preferred embodiment the term "skin" relates to axillary skin. The term "axillary skin" relates to the skin zone of the axle or armpit. The axillary skin typically provides a unique habitat, e.g., for microbes. The axillary skin usually differs from other regions of the body, for instance, with respect to the presence, identity and number of sweat gland. Sweat glands normally form tiny coiled tubes embedded in the dermis or subcutaneous fat. Typically, there are two types of sweat glands: eccrine glands and apocrine glands. Eccrine glands normally produce sweat - i.e. a substance which contains, inter alia, a mixture of water and salts. Typically, sweat plays an important part in regulating the temperature of the body by cooling it, e.g., by evaporation of water from the skin. In addition, sweat may also provide a useful natural method of removing waste products (e.g. toxins) from the body. Typically, the tiny ducts of the eccrine glands pass through the dermis and epidermis and empty directly on to the skin. In general, the number and density of eccrine gland in the axillary skin is higher than in other skin zones of the body. Usually, the sweat production depends on the number of sweat gland, e.g. eccrine glands. This means that the sweat production in or on the axiallary skin is elevated in comparison to other skin zones of the body.

Apocrine glands are typically formed from the same structure as the hair follicle and sebaceous glands. Usually, the apocrine glands become very active with the onset of puberty. Typically, the axillary skin comprises aprocine sweat glands, whereas most of the other skin zones of the body do usually not contain such glands. Preferably, the number of apocrine sweat glands in the axillary skin is higher than in other skin zones of the body, e.g. the genital area.

The term "suppress" in connection with the release of 3-methyl-2-hexenoic acid or its odorous derivatives means that the release of 3-methyl-2-hexenoic acid or its odorous derivatives when contacted with a microorganism according to the invention is stopped or decreased. A "stopped release" means that 3-methyl-2-hexenoic acid or one of its odorous derivatives is not detectable in a mixture containing a microorganism which is capable of releasing 3-methyl-2-hexenoic acid or its odorous derivatives and a microorganism according to the invention in the presence of odorless axillary secret. A "decreased release" means that the amount of 3-methyl-2-hexenoic acid or of one of its odorous derivatives is reduced in a mixture containing a microorganism which is capable of releasing 3-methyl-2-hexenoic acid or its odorous derivatives and a microorganism according to the invention in the presence of odorless axillary secret in comparison to a mixture in which the microorganism according to the invention is not present. The term "reduced" in connection with the release of 3-methyl-2-hexenoic acid or of one of its odorous derivatives means that the amount of 3-methyl-2-hexenoic acid or of one of its odorous derivatives in a mixture containing a microorganism which is capable of releasing 3-methyl-2-hexenoic acid or its odorous derivatives and a microorganism according to the invention in the presence of odorless axillary secret is 95%, 90%,80%, 70%, 60%, 50%, 40%, 30%, 20%, 10%, 5%, more preferably 3% and most preferably 2% of the amount of 3-methyl-2-hexenoic acid or of one of its odorous derivatives present in a mixture in which the microorganism according to the invention is not present.

The capability of a microorganism according to the invention to suppress the release of 3-methyl-2-hexenoic acid can be determined in an assay as described in the following:

Briefly, such an assay comprises the following steps:
- mixing a microorganism which should be tested for its capability to suppress the release of 3-methyl-2-hexenoic acid or its odorous derivatives with a microorganism which is able to release 3-methyl-2-hexenoic acid or an odorous derivative thereof and with an odorless axillary secret;
- incubating the mixture under conditions allowing the release of 3-methyl-2-hexenoic acid or its odorous derivatives;
- extracting short fatty acids form the supernatant of the mixture; and
- detecting odor release by the occurrence of 3-methyl-2-hexenoic acid or its odorous derivatives;

The mixing of the component may be carried out in any suitable proportion and in any suitable buffer, known to the person skilled in the art. In a preferred embodiment a microorganism which is able to release 3-methyl-2-hexenoic acid or an odorous derivative thereof is aerobically cultivated in BHI broth at 37°C. The cultivation may be carried out, e.g., for 20 to 40 h, preferably for 25 to 35 h and even more preferably for 30 h. As volume for the aerobic cultivation any volume suitable can be used, preferably a volume of 1 to 50 ml, more preferably 5 to 40 ml, even more preferably 10 to 30 ml, and most preferably 20 ml is used. The microorganism which is able to release 3-methyl-2-hexenoic acid or an odorous derivative thereof is subsequently separated from the culture medium by any suitable method, e.g. the culture of said microorganism can be centrifuged, for example at 3000 x g for 10 min. As a further step the obtained microorganisms may be washed by any suitable means known to the person skilled in the art, preferably an obtained cell pellet is washed one to several times in a buffer, e.g. a PBS-buffer, pH 7.0. As a further step, the obtained cells may be resuspended in any suitable buffer, known to the person skilled in the art, preferably an obtained cell pellet is resuspended in, e.g. 20 ml of a buffer, for example, a PBS-buffer, pH 7.0.

The microorganism which should be tested for its capability to suppress the release of 3-methy-2-hexenoic acid or its odorous derivatives is cultivated under conditions known by the skilled person to be suitable. Preferably, it is cultivated under anaerobic conditions in, e.g., MRS broth at 37°C. The cultivation may be carried for any time suitable, for instance for 1 to 3 days, preferably for 30 to 60 h, more preferably for 40 to 50 h and even more preferably for 48 h. As volume for the anaerobic cultivation any volume suitable can be used, preferably a volume of 1 to 1000 µl, more preferably of 10 to 500 µl, even more preferably of 100 to 300 µl, and most preferably of 150 µl is used.

Axillary secret may be obtained by any suitable method known to the person skilled in the art, e.g., the harvest of sterile odorless sweat from sterile axilla. The axilla may be cleaned with any suitable cleaning material known to the skilled artisan, for example with PBS buffer containing 0.1 % of Triton X100. After drying, the axilla may be sterilized, e.g. with 70 % ethanol and a clean tissue. After a certain suitable time known to the person skilled in the art, preferably after 2 to 10 h, more preferably 4 to 6 h and even more preferably after 3 h the axillary secretion may be collected, e.g. by washing and rubbing the axilla with, e.g., 4 times 10 % ethanol. Each washing fraction may be collected, preferably in a glass flask and the fractions may be combined and stored at a suitable temperature know to the skilled person, for instance at -20°C. The collection procedure may be repeated until a sufficient amount of secretion extract is collected, for example 200 ml. The diluted odorless axilla secretion may further be concentrated, e.g. in a rotary evaporator. Afterwards the concentrated axillary secretion may further be centrifuged, e.g. at 5000 x g for 10 min.

For the assay washed cells of the microorganism which is able to release 3-methyl-2-hexenoic acid or an odorous derivative thereof, preferably washed cells, are mixed with odorless axillary secret in any suitable proportion known to the person skilled in the art. In a preferred embodiment, 1 to 500 µl of washed cells are used, more preferably, 10 to 200 µl, even more preferably 30 to 100 µl and most preferably 50 µl are used. The concentrated odorless axillary secret may, e.g. be used in an amount of 1 to 1000 µl, preferably of 5 to 500 µl, more preferably of 10 to 250 µl and most preferably of 100 µl. To such a mixture cells of a culture of a microorganism which should be tested for the capability to suppress the release of 3-methyl-2-hexenoic acid or its odorous derivatives may be added in a suitable amount, known to the skilled artisan. Preferably, 1 to 1000 µl are added, more preferably 5 to 500 µl, even more preferably 10 to 250 µl and most preferably 100 µl are added. As a control any suitable buffer or medium, for instance, PBS-buffer or MRS medium in a suitable, corresponding amount may be added to the mixture as characterized herein above. The samples are incubated under conditions allowing the release of 3-methyl-2-hexenoic acid or its odorous derivatives. Such conditions are known by the skilled person. "Conditions allowing the release of 3-methy-2-hexenoic acid or its odorous derivatives" means conditions which are known to the person skilled in the art to allow a microorganism to release 3-methyl-2-hexenoic acid, as can, for example, be verified in a control in which only a microorganism which is able to release 3-methyl-2-hexenoic acid is present, but no microorganism capable of suppressing the release of 3-methyl-2-hexenoic acid or its odorous derivatives. More preferably, the samples are incubated at 37°C under aerobic conditions, for example, for 5 to 30 h, even more preferably 7 to 25 h, 10 to 20 h and most preferably for 16 h. Afterwards the cells may be centrifuged and the supernatant may be acidified, for example with 6 M HCl. Subsequently short chain fatty acids can be extracted with any method known to the person skilled in the art, preferably with 3 x 150 µl CHCl₃. The extract may further be concentrated, e.g. under nitrogen, to a volume of, e.g., 10 µl.

The presence of 3-methyl-2-hexenoic acid or an odorous derivative thereof can be detected by methods known to the person skilled in the art. Preferably, it is determined by GC/MS analysis, e.g. with a Hewlett-Packard GC 5980 series II/MSD 5971 system equipped with a split/splitless injector and a FFAP column. In a preferred embodiment, a small volume, e.g. 1 µl, of an odorous solution or an extract as described herein above may be injected in a GC/MSD equipped, for instance, with a FFAP column, with; e.g. 30 m, 0.25 mm ID, 0.25 µl film thickness, in a splittless mode. Suitable injector and detector temperatures, known to the person skilled in the art, are chosen. Preferably, injector and detector temperatures of 180°C are chosen. For a sensible separation of short fatty acids suitable temperature conditions known to the person skilled in the art are selected. Preferably, the temperature conditions for a sensible separation of short fatty acids may be 2 min at 100°C followed by a ramp to the final temperature of 180°C at 10°C/min. This temperature may be held for 1 to 100 min, preferably for 5 to 50 min and most preferably for 10 min. The column flow may be set according to the conditions known to the person skilled in the art. Preferably, the column few may be set to 0.5mUmin. The identification of 3-methyl-2-hexenoic acid or an odorous derivative thereof may be carried out by comparison of unknown spectra to commercial standards. As an additional identification parameter, for instance, the relative chromatographic retention time can be used. A microorganism is regarded as being able to suppress the release of 3-methyl-2-hexenoic acid or its odorous derivatives if the amount of 3-methyl-2-hexenoic acid or odorous derivatives thereof detected in such an axillary secret based odor release assay with at least one such microorganism is not more than 95%, 90%, 80%, 70%, 60%, 50%, 40%, 30%, 20%, 10%, preferably not more than 5%, more preferably not more than 3% and most preferably not more than 2% of the amount of 3-methyl-2-hexenoic acid or odorous derivatives thereof that is detectable in a mixture in which the microorganism according to the invention is not present. The described assay may also be used to identify microorganisms which are capable of suppressing the release of 3-methyl-2-hexenoic acid or its odorous derivatives.

An alternative in vitro assay for determining the capability to suppress the release of 3-methyl-2-hexenoic acids or its odorous derivatives is based on the use of N-α-lauryl-glutamine as a substrate. N-α-lauryl-glutamine is an artificial substance that is also recognized as substrate for an aminoacylase by those microorganisms that are capable of releasing 3-methyl-2-hexenoic acid or its odorous derivatives. Due to its structural similarity to the natural substrate N-α-lauryl-glutamine can be used to indirectly determine the capability of a microorganism to suppress the release of 3-methyl-2-hexenoic acids or its odorous derivatives. A corresponding assay is based on a comparison between the amount of N-α-lauryl-glutamine before and after the incubation with a microorganism which should be tested for its capability to suppress the release of 3-methyl-2-hexenoic acid or its odorous derivatives. Briefly, such an assay comprises the following steps:
- mixing a microorganism which should be tested for its capability to suppress the release of 3-methyl-2-hexenoic acid or its odorous derivatives with a microorganism which is able to release 3-methyl-2-hexenoic acid or an odorous derivative thereof and with N-α-lauryl-glutamine;
- incubating the mixture under conditions allowing the release of laureate derived from N-α-lauryl-glutamine; and
- analyzing the supernatant of the mixture for the presence of remaining N-α-lauryl-glutamine.

The mixing of the component may be carried out in any suitable proportion and in any suitable buffer, known to the person skilled in the art. In a preferred embodiment a microorganism which is able to release 3-methyl-2-hexenoic acid or an odorous derivative thereof is aerobically cultivated in BHI broth at 37°C. The cultivation may be carried out, e.g., for 20 to 40 h, preferably for 25 to 35 h and even more preferably for 30 h. As volume for the aerobic cultivation any volume suitable can be used, preferably a volume of 1 to 50 ml, more preferably 5 to 40 ml, even more preferably 10 to 30 ml, and most preferably 20 ml is used. The microorganism which is able to release 3-methyl-2-hexenoic acid or an odorous derivative thereof is subsequently separated from the culture medium by any suitable method, e.g. the culture of said microorganism can be centrifuged, for example at 3000 x g for 10 min. As a further step the obtained microorganisms may be washed by any suitable means known to the person skilled in the art, preferably an obtained cell pellet is washed one to several times in a buffer, e.g. a PBS-buffer, pH 7.0. As a further step, the obtained cells may be resuspended in any suitable buffer, known to the person skilled in the art, preferably an obtained cell pellet is resuspended in, e.g. 20 ml of a buffer, for example, a PBS-buffer, pH 7.0.

The microorganism which should be tested for its capability to suppress the release of 3-methy-2-hexenoic acid or its odorous derivatives is cultivated under conditions known by the skilled person to be suitable. Preferably, it is cultivated under anaerobic conditions in, e.g., MRS broth at 37°C. The cultivation may be carried for any time suitable, for instance for 1 to 3 days, preferably for 30 to 60 h, more preferably for 40 to 50 h and even more preferably for 48 h. As volume for the anaerobic cultivation any volume suitable can be used, preferably a volume of 1 to 1000 µl, more preferably of 10 to 500 µl, even more preferably of 100 to 300 µl, and most preferably of 150 µl is used.

For the assay cells of the microorganism which is able to release 3-methyl-2-hexenoic acid or an odorous derivative thereof, preferably washed cells, are mixed with N-α-lauryl-glutamine (e.g.10mg/ml), in any suitable proportion known to the person skilled in the art. In a preferred embodiment, 1 to 500 µl of cells are used, more preferably, 10 to 200 µl, even more preferably 30 to 100 µl and most preferably 50 µl are used. N-α-lauryl-glutamine can be used in any suitable amount known to the person skilled in the art, e.g. in an amount of 0.1 to 100 µl, preferably of 1 to 50 µl, more preferably of 2 to 25 µl and most preferably in an amount of 5 µl. To such a mixture cells of a culture of a microorganism which should be tested for the capability to suppress the release of 3-methyl-2-hexenoic acid or its odorous derivatives may be added in a suitable amount, known to the skilled artisan. Preferably, 1 to 1000 µl are added, more preferably 5 to 500 µl, even more preferably 10 to 250 µl and most preferably 100 µl are added. As a control any suitable buffer or medium, for instance, PBS-buffer or MRS medium in a suitable, corresponding amount may be added to the mixture as characterized herein above. The samples are incubated under conditions allowing the release of 3-methyl-2-hexenoic acid or its odorous derivatives. Such conditions are known to the skilled person. "Conditions allowing the release of 3-methy-2-hexenoic acid or its odorous derivatives" means conditions which are known to the person skilled in the art to allow a microorganism to release 3-methyl-2-hexenoic acid, as can, for example, be verified in a control in which only a microorganism which is able to release 3-methyl-2-hexenoic acid is present, but no microorganism capable of suppressing the release of 3-methyl-2-hexenoic acid or its odorous derivatives. More preferably, the samples are incubated at 37°C under aerobic conditions, for example, for 5 to 30 h, even more preferably 7 to 25 h, 10 to 20 h and most preferably for 16 h.

Afterwards the cells may be separated from the culture medium, e.g. by centrifugation and the supernatant can be analyzed for the presence of remaining N-α-lauryl-glutamine.

The presence of remaining N-α-lauryl-glutamine, i.e non-cleaved N-α-lauryl-glutamine, can be detected by methods known to the person skilled in the art. Preferably, it is determined by HPLC analysis with UV detection at 198 nm, e.g. with an 1100series HPLC system (Agilent Technologies), equipped with a C8 reversed phase column (e.g. Zorbax Eclipse XDB-C8).

A microorganism is regarded as being able to suppress the release of 3-methyl-2-hexenoic acid or its odorous derivatives if the remaining amount of non-cleaved N-α-lauryl-glutamine detected in such an N-α-lauryl-glutamine based assay with at least one such microorganism amounts to at least 10 %, preferably at least 20 %, 30 %, 40 %, 50 %, 60 %, 70 %. 80 %, 90 %, more preferably at least 95 %, even more preferably at least 97 % and most preferably at least 99 % of the amount of N-α-lauryl-glutamine originally applied in the assay.

The described assay may also be used to identify microorganisms which are capable of suppressing the release of 3-methyl-2-hexenoic acid or its odorous derivatives.

In a particularly preferred embodiment the microorgani for use according to the present invention is a microorganism belonging to the group of lactic acid bacteria. The term "microorganism belonging to the group of lactic acid bacteria" encompasses (a) microorganism(s) which belong(s) to bacteria, in particular belonging to gram-positive fermentative eubacteria, more particularly belonging to the family of lactobacteriaceae including lactic acid bacteria. Lactic acid bacteria are from a taxonomical point of view divided up into the subdivisions of Streptococcus, Leuconostoc, Pediococcus and Lactobacillus. The microorganism for use according to the present invention is preferably a Lactobacillus species. Members of the lactic acid bacteria group normally lack porphyrins and cytochromes, do not carry out electron-transport phosphorylation and hence obtain energy only by substrate-level phosphorylation. i.e. in lactic acid bacteria ATP is synthesized through fermentation of carbohydrates. All of the lactic acid bacteria grow anaerobically, however, unlike many anaerobes, most lactic acid bacteria are not sensitive to oxygen and can thus grow in its presence as well as in its absence. Accordingly, the bacteria for use according to the present invention are preferably aerotolerant anaerobic lactic acid bacteria, preferably belonging to the genus of Lactobacillus.

The lactic acid bacteria for use according to the present invention are preferably rod-shaped or spherical, varying from long and slender to short bent rods, are moreover preferably immotile and/or asporogenous and produce lactic acid as a major or sole product of fermentative metabolism. The genus Lactobacillus to which the microorganisms for use according to the present invention belongs in a preferred embodiment is divided up by the following characteristics into three major subgroups, whereby it is envisaged that the Lactobacillus species of the present invention can belong to each of the three major subgroups:
(a) homofermentative lactobacilli
   (i) producing lactic acid, preferably the L-, D- or DL-isomer(s) of lactic acid in an amount of at least 85% from glucose via the Embden-Meyerhof pathway;
   (ii) growing at a temperature of 45°C, but not at a temperature of 15°C;
   (iii) being long-rod shaped; and
   (iv) having glycerol teichoic acid in the cell wall;
(b) homofermantative lactobacilli
   (i) producing lactic acid, preferably the L- or DL-isomer(s) of lactic acid via the Embden-Meyerhof pathway;
   (ii) growing at a temperature of 15°C, showing variable growth at a temperature of 45°C;
   (iii) being short-rod shaped or coryneform; and
   (iv) having ribitol and/or glycerol teichoic acid in their cell wall;
(c) heterofermentative lactobacilli
   (i) producing lactic acid, preferably the DL-isomer of lactic acid in an amount of at least 50% from glucose via the pentose-phosphate pathway;
   (ii) producing carbondioxide and ethanol
   (iii) showing variable growth at a temperature of 15°C or 45°C;
   (iv) being long or short rod shaped; and
   (v) having glycerol teichoic acid in their cell wall.

Based on the above-described characteristics, the microorganisms for use according to the present invention can be classified to belong to the group of lactic acid bacteria, particularly to the genus of Lactobacillus. By using classical systematics, for example, by reference to the pertinent descriptions in "Bergey's Manual of Systematic Bacteriology" (Williams & Wilkins Co., 1984), a microorganism of the present invention can be determined to belong to the genus of Lactobacillus. Alternatively, the microorganisms of the present invention can be classified to belong to the genus of Lactobacillus by methods known in the art, for example, by their metabolic fingerprint, i.e. a comparable overview of the capability of the microorganism(s) of the present invention to metabolize sugars or by other methods described, for example, in Schleifer et al., System. Appl. Microb., 18 (1995), 461-467 or Ludwig et al., System. Appl. Microb., 15 (1992), 487-501. The microorganisms of the present invention are capable of metabolizing sugar sources which are typical and known in the art for microorganisms belonging to the genus of Lactobacillus.

The affiliation of the microorganisms for use according to the present invention to the genus of Lactobacillus can also be characterized by using other methods known in the art, for example, using SDS-PAGE gel electrophoresis of total protein of the species to be determined and comparing them to known and already characterized strains of the genus Lactobacillus. The techniques for preparing a total protein profile as described above, as well as the numerical analysis of such profiles, are well known to a person skilled in the art. However, the results are only reliable insofar as each stage of the process is sufficiently standardized. Faced with the requirement of accuracy when determining the attachment of a microorganism to the genus of Lactobacillus, standardized procedures are regularly made available to the public by their authors such as that of Pot et al., as presented during a "workshop" organized by the European Union, at the University of Ghent, in Belgium, on Sep. 12 to 16, 1994 (Fingerprinting techniques for classification and identification of bacteria, SDS-PAGE of whole cell protein). The software used in the technique for analyzing the SDS-PAGE electrophoresis gel is of crucial importance since the degree of correlation between the species depends on the parameters and algorithms used by this software. Without going into the theoretical details, quantitative comparison of bands measured by a densitometer and normalized by a computer is preferably made with the Pearson correlation coefficient. The similarity matrix thus obtained may be organized with the aid of the UPGMA (unweighted pair group method using average linkage) algorithm that not only makes it possible to group together the most similar profiles, but also to construct dendograms (see Kersters, Numerical methods in the classification and identification of bacteria by electrophoresis, in Computer-assisted Bacterial Systematics, 337-368, M. Goodfellow, A. G. O'Donnell Ed., John Wiley and Sons Ltd, 1985).

Alternatively, the affiliation of said microorganisms for use according to the present invention to the genus of Lactobacillus can be characterized with regard to ribosomal RNA in a so called Riboprinter.RTM. More preferably, the affiliation of the newly identified species for use according to the invention to the genus Lactobacillus is demonstrated by comparing the nucleotide sequence of the 16S ribosomal RNA of the bacteria of the invention, or of their genomic DNA which codes for the 16S ribosomal RNA, with those of other genera and species of lactic acid bacteria known to date. Another preferred alternative for determining the attachment of the newly identified species of the invention to the genus Lactobacillus is the use of species-specific PCR primers that target the 16S-23S rRNA spacer region. Another preferred alternative is RAPD-PCR (Nigatu et al. in Antonie van Leeuwenhoek (79), 1-6, 2001) by virtue of that a strain specific DNA pattern is generated which allows to determine the affiliation of an identified microorganisms in accordance with the present invention to the genus of Lactobacillus. Further techniques useful for determining the affiliation of the microorganism of the present invention to the genus of Lactobacillus are restriction fragment length polymorphism (RFLP) (Giraffa et al., Int. J. Food Microbiol. 82 (2003), 163-172), fingerprinting of the repetitive elements (Gevers et al., FEMS Microbiol. Lett. 205 (2001) 31-36) or analysis of the fatty acid methyl ester (FAME) pattern of bacterial cells (Heyrman et al., FEMS Microbiol. Lett. 181 (1991), 55-62). Alternatively, lactobacilli can be determined by lectin typing (Annuk et al., J. Med. Microbiol. 50 (2001), 1069-1074) or by analysis of their cell wall proteins (Gatti et al., Lett. Appl. Microbiol. 25 (1997), 345-348.

In a preferred embodiment of the present application the microorganism is a probiotic microorganism. The term "probiotic" in the context of the present invention means that the microorganism has a beneficial effect on health if it is topically applied to the skin. Preferably, a "probiotic" microorganism is a live microorganism which, when topically applied to the skin, e.g. of the axilla, is beneficial for health of this tissue. Most preferably this means that the microorganism has a positive effect on the micro flora of the skin.

In a preferred embodiment the microorganism for use according to the present invention belongs to the species of *Lactobacillus plantarum, Lactobacillus crispatus, Lactobacillus acidophilus II, Lactobacillus acidophilus III* or *Lactobacillus delbrückii delbrückii.* However, the Lactobacillus species are not limited thereto.

The microorganism for use according to the the present invention is selected from the group consisting of *Lactobacillus plantarum, Lactobacillus crispatus, Lactobacillus acidophilus* II, *Lactobacillus acidophilus III* or *Lactobacillus delbrückil delbrockii* being deposited at the DSMZ by the OrganoBalance GmbH, Gustav-Meyer-Allee 25, 13355 Berlin, Germany under the accession number DSM 17598 (Lactobacillus plantarum, OB-AG-0002), DSM 17567 (Lactobacillus crispatus, OB-AG-0003), DSM 17568 (Lactobacillus acidophilus II, OB-AG-0004), DSM 17569 (Lactobacillus acidophilus II, OB-AG-0005), DSM 17570 (Lactobacillus acidophilus III, OB-AG-0006) and DSM 17571 (Lactobacillus delbrückii delbrückii, OB-AG-0007).

The invention also relates to a mutant of the above-mentioned deposited Lactobacillus strain wherein said mutants have retained the capability to suppress the release of 3-methyl-2-hexenoic acid or odorous derivatives thereof.

The term "*Lactobacillus plantarum, Lactobacillus crispatus, Lactobacillus acidophilus II, Lactobacillus acidophilus III* or *Lactobacillus delbrückii delbrückii* being deposited at the DSMZ" relates to cells of a microorganism belonging to the species *Lactobacillus plantarum, Lactobacillus crispatus, Lactobacillus acidophilus II, Lactobacillus acidophilus III* or *Lactobacillus delbrückii delbrückii* deposited at the Deutsche Sammlung für Mikroorganismen und Zellkulturen (DSMZ) on September 12, 2005 by the OrganoBalance GmbH, Gustav-Meyer-Allee 25, 13355 Berlin, Germany and having the following deposit numbers: DSM 17598 (Lactobacillus plantarum, OB-AG-0002), DSM 17567 (Lactobacillus crispatus, OB-AG-0003), DSM 17568 (Lactobacillus acidophilus II, OB-AG-0004), DSM 17569 (Lactobacillus acidophilus II, OB-AG-0005), DSM 17570 (Lactobacillus acidophilus III, OB-AG-0006) and DSM 17571 (Lactobacillus delbrückii delbrückii, OB-AG-0007). The DSMZ is located at the Mascheroder Weg 1b, D-38124 Braunschweig, Germany. The aforementioned deposits were made pursuant to the terms of the Budapest treaty on the international recognition of the deposit of microorganisms for the purposes of patent procedures.

In a particular preferred embodiment the microorganisms for use according to the present invention are "isolated" or "purified". The term "isolated" means that the material is removed from its original environment, e.g. the natural environment if it is naturally occurring, or the culture medium if it is cultured. For example, a naturally-occurring microorganism, preferably a Lactobacillus species, separated from some or all of the coexisting materials in the natural system, is isolated. Such a microorganism could be part of a composition, and is to be regarded as still being isolated in that the composition is not part of its natural environment.

The term "purified" does not require absolute purity; rather, it is intended as a relative definition. Individual microorganisms obtained from a library have been conventionally purified to microbiological homogeneity, i.e. they grow as single colonies when streaked out on agar plates by methods known in the art. Preferably, the agar plates that are used for this purpose are selective for Lactobacillus species. Such selective agar plates are known in the art.

In another aspect the present invention relates to the use of an inactivated form of the microorganism of the present invention, which is, e.g., thermally inactivated or lyophilized, but which retains the property of retained their capability to suppress the release of 3-methyl-2-hexenoic acid or odorous derivatives thereof.

According to the present invention the term "inactivated form of the microorganism of the present invention" includes a dead or inactivated cell of the microorganism of the present invention, preferably of the Lactobacillus species disclosed herein, which is no longer capable to form a single colony on a plate specific for microorganisms belonging to the genus of Lactobacillus. Said dead or inactivated cell may have either an intact or broken cell membrane. Methods for killing or inactivating cells of the microorganism of the present invention are known in the art. El-Nezami et al., J. Food Prot. 61 (1998), 466-468 describes a method for inactivating Lactobacillus species by UV-irradiation. Preferably, the cells of the microorganism of the present invention are thermally inactivated or lyophilised. Lyophilisation of the cells of the present invention has the advantage that they can be easily stored and handled while retaining their property to suppress the release of 3-methyl-2-hexenoic acid or odorous derivatives thereof.

Moreover, lyophilised cells can be grown again when applied under conditions known in the art to appropriate liquid or solid media. Lyophilization is done by methods known in the art. Preferably, it is carried out for at least 2 hours at room temperature, i.e. any temperature between 16°C and 25°C. Moreover, the lyophilized cells of the microorganism of the present invention are stable for at least 4 weeks at a temperature of 4°C so as to still retain their properties as described above. Thermal inactivation can be achieved by incubating the cells of the microorganism of the present invention for at least 2 hours at a temperature of 170°C. Yet, thermal inactivation is preferably achieved by autoclaving said cells at a temperature of 121°C for at least 20 minutes in the presence of saturated steam at an atmospheric pressure of 2 bar. In the alternative, thermal inactivation of the cells of the microorganism of the present invention is achieved by freezing said cells for at least 4 weeks, 3 weeks, 2 weeks, 1 week, 12 hours, 6 hours, 2 hours or 1 hour at -20°C. It is preferred that at least 70%, 75% or 80%, more preferably 85%, 90% or 95% and particularly preferred at least 97%, 98%, 99% and more particularly preferred, 99.1%, 99.2%, 99.3%, 99.4%, 99.5%, 99.6%, 99.7%, 99.8% or 99.9% and most particularly preferred 100% of the cells of the inactivated form of the microorganism of the present invention are dead or inactivated, however, they have still the capability to suppress the release of 3-methyl-2-hexenoic acid or odorous derivatives thereof. Whether the inactivated form of the microorganism of the present invention is indeed dead or inactivated can be tested by methods known in the art, for example, by a test for viability.

The term "inactivated form of the microorganism of the present invention" also encompasses lysates or fractions of the microorganism of the present invention, preferably of the Lactobacillus species disclosed herein, wherein said lysates or fractions preferably suppress the release of 3-methyl-2-hexenoic acid or odorous derivatives thereof. This suppression can be tested as described herein and in particular as described in the appended Examples. In case, a lysate or fraction of the microorganism of the present invention may suppress the release of 3-methyl-2-hexenoic acid or odorous derivatives thereof then the skilled person can, for example, further purify said lysate or fraction by methods known in the art, which are exemplified herein below, so as to remove substances which may suppress the release of 3-methyl-2-hexenoic acid odorous derivatives thereof. This means that, if a lysate or fraction of the microorganism of the present invention may not suppress the release of 3-methyl-2-hexenoic acid or odorous derivatives thereof, the skilled person can, for example, further purify said lysate or fraction by methods known in the art so as to remove substances which may impede the suppression of the release of 3-methyl-2-hexenoic acid odorous derivatives thereof. Afterwards the person skilled in the art can again test said lysate or fraction whether it suppresses the release of 3-methyl-2-hexenoic acid or odorous derivatives thereof.

According to the present invention the term "lysate" means a solution or suspension in an aqueous medium of cells of the microorganism of the present invention that are broken or an extract. However, the term should not be construed in any limiting way. The cell lysate comprises, e.g., macromolecules, like DNA, RNA, proteins, peptides, carbohydrates, lipids and the like and/or micromolecules, like amino acids, sugars, lipid acids and the like, or fractions of it. Additionally, said lysate comprises cell debris which may be of smooth or granular structure. Methods for preparing cell lysates of microorganism are known in the art, for example, by employing French press, cells mill using glass or iron beads or enzymatic cell lysis and the like. In addition, lysing cells relates to various methods known in the art for opening/destroying cells. The method for lysing a cell is not important and any method that can achieve lysis of the cells of the microorganism of the present invention may be employed. An appropriate one can be chosen by the person skilled in the art, e.g. opening/destruction of cells can be done enzymatically, chemically or physically. Non-limiting examples for enzymes and enzyme cocktails are proteases, like proteinase K, lipases or glycosidases; non-limiting examples for chemicals are ionophores, detergents, like sodium dodecyl sulfate, acids or bases; and non-limiting examples of physical means are high pressure, like French-pressing, osmolarity, temperature, like heat or cold. Additionally, a method employing an appropriate combination of an enzyme other than the proteolytic enzyme, an acid, a base and the like may also be utilized. For example, the cells of the microorganism of the present invention are lysed by freezing and thawing, more preferably freezing at temperatures below -70°C and thawing at temperatures of more than 30°C, particularly freezing is preferred at temperatures below -75°C and thawing is preferred at temperatures of more than 35°C and most preferred are temperatures for freezing below -80°C and temperatures for thawing of more than 37°C. It is also preferred that said freezing/thawing is repeated for at least 1 time, more preferably for at least 2 times, even more preferred for at least 3 times, particularly preferred for at least 4 times and most preferred for at least 5 times.

Accordingly, those skilled in the art can prepare the desired lysates by referring to the above general explanations, and appropriately modifying or altering those methods, if necessary. Preferably, the aqueous medium used for the lysates as described is water, physiological saline, or a buffer solution. An advantage of a bacterial cell lysate is that it can be easily produced and stored cost efficiently since less technical facilities are needed.

Preferably, the term "extract" means a subcellular component of the microorganism of the present invention, e.g., macromolecules, like DNA, RNA, proteins, peptides, carbohydrates, lipids and the like and/or micromolecules, like amino acids, sugars, lipid acids and the like or any other organic compound or molecule, or fractions of it, wherein said extract preferably suppresses the release of 3-methyl-2-hexenoic acid or odorous derivatives thereof. More preferably, the term "extract" refers to any of the above described subcellular components in a cell-free medium.

In a further preferred embodiment an extract may be obtained by lysing cells according to various methods known in the art for opening/destroying cells, as described herein above and/or as supernatant of a centrifugation procedure of a culture of the microorganism of the present invention in any appropriate liquid, medium or buffer known to the person skilled in the art or of a lysate of such a culture or any other suitable cell suspension. More preferably, the extract may be a purified lysate or cell culture supernatant or any fraction or subportion thereof, wherein said purified lysate or cell culture supernatant or any fraction or subportion thereof suppresses the release of 3-methyl-2-hexenoic acid or odorous derivatives thereof. Suitable methods for purification of an extract are known to the person skilled in the art and comprise, for example, affinity chromatography, ion-exchange chromatography, size-exclusion chromatography, reversed phase-chromatography, and chromatography with other chromatographic material in column or batch methods, other fractionation methods, e.g., filtration methods, e.g., ultrafiltration, dialysis, dialysis and concentration with size-exclusion in centrifugation, centrifugation in density-gradients or step matrices, precipitation, e.g., affinity precipitations, salting-in or salting-out (ammoniumsulfate-precipitation), alcoholic precipitations or other proteinchemical, molecular biological, biochemical, immunological, chemical or physical.

According to the invention, lysates are also preparations of fractions of molecules from the above-mentioned lysates. These fractions can be obtained by methods known to those skilled in the art, e.g., chromatography, including, e.g., affinity chromatography, ion-exchange chromatography, size-exclusion chromatography, reversed phase-chromatography, and chromatography with other chromatographic material in column or batch methods, other fractionation methods, e.g., filtration methods, e.g., ultrafiltration, dialysis, dialysis and concentration with size-exclusion in centrifugation, centrifugation in density-gradients or step matrices, precipitation, e.g., affinity precipitations, salting-in or salting-out (ammoniumsulfate-precipitation), alcoholic precipitations or other proteinchemical, molecular biological, biochemical, immunological, chemical or physical methods to separate above components of the lysates. In a preferred embodiment those fractions which are more immunogenic than others are preferred. Those skilled in the art are able to choose a suitable method and determine its immunogenic potential by referring to the above general explanations and specific explanations in the examples herein, and appropriately modifying or altering those methods, if necessary.

Accordingly, the term "an inactive form of the microorganism of the present invention" also encompasses filtrates of the microorganism of the present invention, preferably of the Lactobacillus species disclosed herein, wherein said filtrates preferably suppress the release of 3-methyl-2-hexenoic acid or odorous derivatives thereof. This inhibition can be tested as described herein and in particular as described in the appended Examples. In case, a filtrate of the microorganism of the present invention may not suppress the release of 3-methyl-2-hexenoic acid or odorous derivatives thereof, then the skilled person can, for example, further purify said filtrate by methods known in the art, so as to remove substances which may impede the suppression of the release of 3-methyl-2-hexenoic acid odorous derivatives thereof. Afterwards the person skilled in the art can again test said filtrate whether it suppresses the release of 3-methyl-2-hexenoic acid or odorous derivatives thereof.

The term "filtrate" means a cell-free solution or suspension of the microorganism of the present invention which has been obtained as supernatant of a centrifugation procedure of a culture of the microorganism of the present invention in any appropriate liquid, medium or buffer known to the person skilled in the art. However, the term should not be construed in any limiting way. The filtrate comprises, e.g., macromolecules, like DNA, RNA, proteins, peptides, carbohydrates, lipids and the like and/or micromolecules, like amino acids, sugars, lipid acids and the like, or fractions of it. Methods for preparing filtrates of microorganism are known in the art. In addition, "filtrate" relates to various methods known in the art. The exact method is not important and any method that can achieve filtration of the cells of the microorganism of the present invention may be employed.

The term "an inactive form of the microorganism of the present invention" encompasses any part of the cells of the microorganism of the present invention. Preferably, said inactive form is a membrane fraction obtained by a membrane-preparation. Membrane preparations of microorganisms belonging to the genus of Lactobacillus can be obtained by methods known in the art, for example, by employing the method described in Rollan et al., Int. J. Food Microbiol. 70 (2001), 303-307, Matsuguchi et al., Clin. Diagn. Lab. Immunol. 10 (2003), 259-266 or Stentz et al., Appl. Environ. Microbiol. 66 (2000), 4272-4278 or Varmanen et al., J. Bacteriology 182 (2000), 146-154. Alternatively, a whole cell preparation is also envisaged.

In another aspect the present invention relates to a composition comprising a microorganism according to the present invention or a mutant, derivative or inactive form of this microorganism as described above. In a preferred embodiment, said composition comprises a microorganism as described above in an amount between 10² to 10¹² cells, preferably 10³ to 10⁸ cells per mg in a solid form of the composition. In case of a liquid form of compositions, the amount of the microorganisms is between 10² to 10¹³ cells per ml. In a further preferred embodiment said compositions are in the form of emulsions, e.g. oil in water or water in oil emulsions, in the form of ointments or in the form of micro-capsules. In case of emulsions, ointments or microcapsules the compositions comprise a microorganism as described herein in an amount between 10² to 10¹³ cells per ml. However, for specific compositions the amount of the microorganism may be different as is described herein.

The term "composition" also includes textile compositions as described further below.

In a still further aspect, the present disclosure provides a method for the production of a composition for suppressing the release of 3-methyl-2-hexenoic acid by axillary bacteria comprising the steps of formulating a microorganism according to the invention or a mutant, derivative or inactive form of this microorganism as described above with a cosmetically or pharmaceutical acceptable carrier or excipient.

The term "composition", as used in accordance with the present invention, relates to (a) composition(s) which comprise(s) at least one microorganism of the present invention or mutant, derivative or inactive form of said microorganism as described above. It is envisaged that the compositions of the present invention which are described herein below comprise the aforementioned ingredients in any combination. It may, optionally, comprise at least one further ingredient suitable for suppressing the release of 3-methyl-2-hexenoic acid or its odorous derivatives by axillary bacteria. Accordingly, it may optionally comprise any combination of the hereinafter described further ingredients. The term "ingredient suitable for suppressing the release of 3-methyl-2-hexenoic acid or its odorous derivatives by axillary bacteria" encompasses compounds or compositions and/or combinations thereof which lead to an increased pH value.

The composition may be in solid, liquid or gaseous form and may be, inter alia, in the form of (a) powder(s), (a) solution(s) (an) aerosol(s), suspensions, emulsions, liquids, elixirs, extracts, tincture or fluid extracts or in a form which is particularly suitable for topical administration. Forms suitable for topical application include, e.g., a deodorant, a paste, an ointment, a pumpspray, a lotion, a gel, a cream, a cream or fluid gel distributed as an aerosol spray, e.g. in a pump-dispenser bottle or as a roll-on, in the form of thick creams distributed in tubes or a grille, in the form of wands, or as a transdermal patch.

Preferably, the composition of the present invention is a cosmetic composition further comprising a cosmetically acceptable carrier or excipient.

The cosmetic composition of the present invention comprises the microorganism of the present invention, mutant, derivative or inactive form thereof as described above in connection with the composition of the invention and further a cosmetically acceptable carrier. Preferably the cosmetic composition of the present invention is for use in topical applications.

The term "cosmetically acceptable carrier" as used herein means a suitable vehicle, which can be used to apply the present compositions to the skin in a safe and effective manner. Such vehicle may include materials such as emulsions, e.g. oil in water or water in oil emulsions, ointments or micro-capsules. The term "safe and effective manner" as used herein, means a sufficient amount to suppress the release of 3-methyl-2-hexenoic acid.

In another aspect the present invention relates to a pharmaceutical composition comprising the microorganism of the present invention or a derivative or mutant or an inactive form thereof as described above further comprising a pharmaceutical acceptable carrier or excipient. The pharmaceutical composition preferably is in a form which is suitable for topical administration.

The term "carrier" refers to a diluent, adjuvant, excipient, or vehicle with which the therapeutic is administered. Such a carrier is pharmaceutically acceptable, i.e. is non-toxic to a recipient at the dosage and concentration employed. It is preferably isotonic, hypotonic or weakly hypertonic and has a relatively low ionic strength, such as provided by a sucrose solution. Such pharmaceutical carriers can be sterile liquids, such as water and oils, including those of petroleum, animal, vegetable or synthetic origin, such as peanut oil, soybean oil, mineral oil, sesame oil and the like. Saline solutions and aqueous dextrose and glycerol solutions can also be employed as liquid carriers. Suitable pharmaceutical excipients include starch, glucose, sucrose, gelatin, malt, rice, flour, chalk, silica gel, sodium stearate, glycerol monostearate, talc, sodium ion, dried skim milk, glycerol, propylene, glycol, water, ethanol and the like. The composition, if desired, can also contain minor amounts of wetting or emulsifying agents, or pH buffering agents. These compositions can take the form of, e.g., solutions, suspensions, emulsion, powders, sustained-release formulations and the like. Examples of suitable pharmaceutical carriers are described in "Remington's Pharmaceutical Sciences" by E.W. Martin. Some other examples of substances which can serve as pharmaceutical carriers are sugars, such as glucose and sucrose; starches such as corn starch and potato starch; cellulose and its derivatives such as sodium carboxymethycellulose, ethylcellulose and cellulose acetates; powdered tragancanth; malt; gelatin; talc; stearic acids; magnesium stearate; calcium sulfate; calcium carbonate; vegetable oils, such as peanut oils, cotton seed oil, sesame oil, olive oil, corn oil and oil of theobroma; polyols such as propylene glycol, glycerine, sorbitol, manitol, and polyethylene glycol; agar; alginic acids; pyrogen-free water; isotonic saline; cranberry extracts and phosphate buffer solution; skim milk powder; as well as other non-toxic compatible substances used in pharmaceutical formulations such as Vitamin C, estrogen and echinacea, for example. Wetting agents and lubricants such as sodium lauryl sulfate, as well as coloring agents, flavoring agents, lubricants, excipients, tabletting agents, stabilizers, anti-oxidants and preservatives, can also be present. It is also advantageous to administer the active ingredients in encapsulated form, e.g. as cellulose encapsulation, in gelatine, with polyamides, niosomes, wax matrices, with cyclodextrins or liposomally encapsulated.

Generally, the ingredients are supplied either separately or mixed together in unit dosage form, for example, as a dry lyophilised powder or water free concentrate in a hermetically sealed container such as an ampoule or sachette indicating the quantity of active agent.

The pharmaceutical composition of the invention can be formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

An in vitro assay, e.g. one of those described in the Examples, may optionally be employed to help identify optimal dosage ranges. The precise dose to be employed in the formulation will also depend on the route of administration, and the seriousness of the disease or disorder, and should be decided according to the judgment of the practitioner and each patient's circumstances. The topical route of administration is preferred. Effective doses may be extrapolated from dose-response curves derived from in vitro or (animal) model test systems. Preferably, the pharmaceutical composition is administered directly or in combination with an adjuvant. Adjuvants may be selected from the group consisting of a chloroquine, protic polar compounds, such as propylene glycol, polyethylene glycol, glycerol, EtOH, 1-methyl L-2-pyrrolidone or their derivatives, or aprotic polar compounds such as dimethylsulfoxide (DMSO), diethylsulfoxide, di-n-propylsulfoxide, dimethylsulfone, sulfolane, dimethylformamide, dimethylacetamide, tetramethylurea, acetonitrile or their derivatives. These compounds are added in conditions respecting pH limitations. The composition of the present invention can be administered to a vertebrate. "Vertebrate" as used herein is intended to have the same meaning as commonly understood by one of ordinary skill in the art. Particularly, "vertebrate" encompasses mammals, and more particularly humans.

The term "administered" means administration of a therapeutically effective dose of the aforementioned composition. By "therapeutically effective amount" is meant a dose that produces the effects for which it is administered, preferably this effect is the suppression of the release of 3-methyl-2-hexenoic acid or its odorous derivatives by axillary bacteria. The exact dose will depend on the purpose of the treatment, and will be ascertainable by one skilled in the art using known techniques. As is known in the art and described above, adjustments for systemic versus localized delivery, age, body weight, general health, sex, diet, time of administration, drug interaction and the severity of the condition may be necessary, and will be ascertainable with routine experimentation by those skilled in the art.

The methods are applicable to human therapy. The compounds described herein having the desired therapeutic activity may be administered in a physiologically acceptable carrier to a patient, as described herein. Depending upon the manner of administration, the compounds may be formulated in a variety of ways as discussed below. The concentration of the therapeutically active compound in the formulation may vary from about 0.01-100 wt %. The agent may be administered alone or in combination with other treatments.

The administration of the pharmaceutical composition can be done in a variety of ways. The preferable route of administering is the topical route.

The attending physician and clinical factors will determine the dosage regimen. As is well known in the medical arts, dosages for any one patient depends upon many factors, including the patient's size, body surface area, age, the particular compound to be administered, sex, time and route of administration, general health, and other drugs being administered concurrently. A typical dose can be, for example, in the range of 0.001 to 1000 µg; however, doses below or above this exemplary range are envisioned, especially considering the aforementioned factors.

The dosages are preferably given once a week, more preferably 2 times, 3 times, 4 times, 5 times or 6 times a week and most preferably daily and even more preferably, 2 times a day or more often. However, during progression of the treatment the dosages can be given in much longer time intervals and in need can be given in much shorter time intervals, e.g., several times a day. In a preferred case the immune response is monitored using herein described methods and further methods known to those skilled in the art and dosages are optimized, e.g., in time, amount and/or composition. Progress can be monitored by periodic assessment. It is also envisaged that the pharmaceutical compositions are employed in co-therapy approaches, i.e. in co-administration with other medicaments or drugs, for example other drugs for suppressing the release of 3-methyl-2-hexenoic acid or its odorous derivatives.

Topical administration of the cosmetic or pharmaceutical composition of the present invention is useful when the desired treatment involves areas or organs readily accessible by topical administration. For application topically to the skin, the pharmaceutical composition is preferably formulated in the form of a deodorant or a spray (pumpspray or aerosol) or with a paste, an ointment, a lotion, a cream, a gel or a transdermal patch. The cosmetic or pharmaceutical preparations can, depending on the field of use, also be in the form of a foam, gel spray, mousse, suspensions or powders.

The cosmetic or pharmaceutical composition may also be formulated in the form of spray (pumpspray or aerosol). Suitable propellants for aerosols according to the invention are the customary propellants, for example propane, butane, pentane and others.

Alternatively the cosmetic or pharmaceutical composition may also be formulated with a suitable paste comprising the active ingredient suspended in a carrier. Such carriers include, but are not limited to, petroleum, soft white paraffin, yellow petroleum jelly and glycerol.

The cosmetic or pharmaceutical composition may also be formulated with a suitable ointment comprising the active components suspended or dissolved in a carrier. Such carriers include, but are not limited to, one or more of glycerol, mineral oil, liquid oil, liquid petroleum, white petroleum, yellow petroleum jelly, propylene glycol, alcohols, triglycerides, fatty acid esters such as cetyl ester, polyoxyethylene polyoxypropylene compound, waxes such as white wax and yellow beeswax, fatty acid alcohols such as cetyl alcohol, stearyl alcohol and cetylstearylalcohol, fatty acids such as stearic acid, cetyl stearate, lanolin, magnesium hydroxide, kaolin and water. Alternatively, the cosmetic or pharmaceutical composition may also be formulated with a suitable lotion or cream comprising the active components suspended or dissolved in a carrier. Such carriers include, but are not limited to, one or more of mineral oil such as paraffin, vegetable oils such as castor oil, castor seed oil and hydrogenated castor oil, sorbitan monostearat, polysorbat, fatty acid esters such as cetyl ester, wax, fatty acid alcohols such as cetyl alcohol, stearyl alcohol, 2-octyldodecanol, benzyl alcohol, alcohols, triglycerides and water.

Alternatively, the cosmetic or pharmaceutical composition may also be formulated with a suitable gel comprising the active components suspended or dissolved in a carrier. Such carriers include, but are not limited to, one or more of water, glycerol, propyleneglycole, liquid paraffin, polyethylene, fatty oils, cellulose derivatives, bentonite and colloidal silicon dioxide.

The preparations according to the invention may generally comprise further auxiliaries as are customarily used in such preparations, e.g. preservatives, perfumes, antifoams, dyes, pigments, thickeners, surface-active substances, emulsifiers, emollients, finishing agents, fats, oils, waxes or other customary constituents, of a cosmetic or dermatological formulation, such as alcohols, polyols, polymers, foam stabilizers, solubility promoters, electrolytes, organic acids, organic solvents, or silicone derivatives.

The cosmetic or pharmaceutical composition according to the invention may comprise emollients. Emollients may be used in amounts which are effective to prevent or relieve dryness. Useful emollients include, without limitation: hydrocarbon oils and waxes; silicone oils; triglyceride esters; acetoglyceride esters; ethoxylated glyceride; alkyl esters; alkenyl esters; fatty acids; fatty alcohols; fatty alcohol ethers; etheresters; lanolin and derivatives; polyhydric alcohols (polyols) and polyether derivatives; polyhydric alcohol (polyol) esters; wax esters; beeswax derivatives; vegetable waxes; phospholipids; sterols; and amides.

Thus, for example, typical emollients include mineral oil, especially mineral oils having a viscosity in the range of 50 to 500 SUS, lanolin oil, mink oil, coconut oil, cocoa butter, olive oil, almond oil, macadamia nut oil, aloa extract, jojoba oil, safflower oil, corn oil, liquid lanolin, cottonseed oil, peanut oil, purcellin oil, perhydrosqualene (squalene), caster oil, polybutene, odorless mineral spirits, sweet almond oil, avocado oil, calophyllum oil, ricin oil, vitamin E acetate, olive oil, mineral spirits, cetearyl alcohol (mixture of fatty alcohols consisting predominantly of cetyl and stearyl alcohols), linolenic alcohol, oleyl alcohol, octyl dodecanol, the oil of cereal germs such as the oil of wheat germ cetearyl octanoate (ester of cetearyl alcohol and 2-ethylhexanoic acid), cetyl palmitate, diisopropyl adipate, isopropyl palmitate, octyl palmitate, isopropyl myristate, butyl myristate, glyceryl stearate, hexadecyl stearate, isocetyl stearate, octyl stearate, octylhydroxy stearate, propylene glycol stearate, butyl stearate, decyl oleate, glyceryl oleate, acetyl glycerides, the octanoates and benzoates of (C12-C15) alcohols, the octanoates and decanoates of alcohols and polyalcohols such as those of glycol and glycerol, and ricin- oleates of alcohols and poly alcohols such as those of isopropyl adipate, hexyl laurate, octyl dodecanoate, dimethicone copolyol, dimethiconol, lanolin, lanolin alcohol, lanolin wax, hydrogenated lanolin, hydroxylated lanolin, acetylated lanolin, petrolatum, isopropyl lanolate, cetyl myristate, glyceryl myristate, myristyl myristate, myristyl lactate, cetyl alcohol, isostearyl alcohol stearyl alcohol, and isocetyl lanolate, and the like.

Moreover, the cosmetic or pharmaceutical composition according to the invention may also comprise emulsifiers. Emulsifiers (i.e., emulsifying agents) are preferably used in amounts effective to provide uniform blending of ingredients of the composition. Useful emulsifiers include (i) anionics such as fatty acid soaps, e.g., potassium stearate, sodium stearate, ammonium stearate, and triethanolamine stearate; polyol fatty acid monoesters containing fatty acid soaps, e.g., glycerol monostearate containing either potassium or sodium salt; sulfuric esters (sodium salts), e.g., sodium lauryl 5 sulfate, and sodium cetyl sulfate; and polyol fatty acid monoesters containing sulfuric esters, e.g., glyceryl monostearate containing sodium lauryl surfate; (ii) cationics chloride such as N(stearoyl colamino formylmethyl) pyridium; N-soya-N-ethyl morpholinium ethosulfate; alkyl dimethyl benzyl ammonium chloride; diisobutylphenoxytheoxyethyl dimethyl benzyl ammonium chloride; and cetyl pyridium chloride; and (iii) nonionics such as polyoxyethylene fatty alcohol ethers, e.g., monostearate; polyoxyethylene lauryl alcohol; polyoxypropylene fatty alcohol ethers, e.g., propoxylated oleyl alcohol; polyoxyethylene fatty acid esters, e.g., polyoxyethylene stearate; polyoxyethylene sorbitan fatty acid esters, e.g., polyoxyethylene sorbitan monostearate; sorbitan fatty acid esters, e.g., sorbitan; polyoxyethylene glycol fatty acid esters, e.g., polyoxyethylene glycol monostearate; and polyol fatty acid esters, e.g., glyceryl monostearate and propylene glycol monostearate; and ethoxylated lanolin derivatives, e.g., ethoxylated lanolins, ethoxylated lanolin alcohols and ethoxylated cholesterol. The selection of emulsifiers is exemplarly described in Schrader, Grundlagen und Rezepturen der Kosmetika, Hüthig Buch Verlag, Heidelberg, 2nd edition, 1989, 3rd part.

The cosmetic or pharmaceutical composition according to the invention may also include a surfactant. Suitable surfactants may include, for example, those surfactants generally grouped as cleansing agents, emulsifying agents, foam boosters, hydrotropes, solubilizing agents, suspending agents and nonsurfactants (facilitates the dispersion of solids in liquids).

The surfactants are usually classified as amphoteric, anionic, cationic and nonionic surfactants. Amphoteric surfactants include acylamino acids and derivatives and N-alkylamino acids. Anionic surfactants include: acylamino acids and salts, such as, acylglutamates, acylpeptides, acylsarcosinates, and acyltaurates; carboxylic acids and salts, such as, alkanoic acids, ester carboxylic acids, and ether carboxylic acids; sulfonic acids and salts, such as, acyl isethionates, alkylaryl sulfonates, alkyl sulfonates, and sulfosuccinates; sulfuric acid esters, such as, alkyl ether sulfates and alkyl sulfates. Cationic surfactants include: alkylamines, alkyl imidazolines, ethoxylated amines, and quaternaries (such as, alkylbenzyldimethylammonium salts, alkyl betaines, heterocyclic ammonium salts, and tetra alkylammonium salts). And nonionic surfactants include: alcohols, such as primary alcohols containing 8 to 18 carbon atoms; alkanolamides such as alkanolamine derived amides and ethoxylated amides; amine oxides; esters such as ethoxylated carboxylic acids, ethoxylated glycerides, glycol esters and derivatives, monoglycerides, polyglyceryl esters, polyhydric alcohol esters and ethers, sorbitan/sorbitol esters, and triesters of phosphoric acid; and ethers such as ethoxylated alcohols, ethoxylated lanolin, ethoxylated polysiloxanes, and propoxylated polyoxyethylene ethers.

Furthermore, a cosmetic or pharmaceutical composition according to the invention may also comprise a film former. Suitable film formers which are used in accord with the invention keep the composition smooth and even and include, without limitation: acrylamide/sodium acrylate copolymer; ammonium acrylates copolymer; Balsam Peru; cellulose gum; ethylene/maleic anhydride copolymer; hydroxyethylcellulose; hydroxypropylcellulose; polyacrylamide; polyethylene; polyvinyl alcohol; pvm/MA copolymer (polyvinyl methylether/maleic anhydride); PVP (polyvinylpyrrolidone); maleic anhydride copolymer such as PA-18 available from Gulf Science and Technology; PVP/hexadecene copolymer such as Ganex V-216 available from GAF Corporation; acryliclacrylate copolymer; and the like.

Generally, film formers can be used in amounts of about 0.1% to about 10% by weight of the total composition with about 1% to about 8% being preferred and about 0.1 DEG/O to about 5% being most preferred. Humectants can also be used in effective amounts, including: fructose; glucose; glulamic acid; glycerin; honey; maltitol; methyl gluceth-10; methyl gluceth-20; propylene glycol; sodium lactate; sucrose; and the like.

Of course, the cosmetic or pharmaceutical composition of the present invention can also comprise a preservative. Preservatives according to certain compositions of the invention include, without limitation: butylparaben; ethylparaben; imidazolidinyl urea; methylparaben; O-phenylphenol; propylparaben; quatemium-14; quaternium-15; sodium dehydroacetate; zinc pyrithione; and the like.

The preservatives are used in amounts effective to prevent or retard microbial growth. Generally, the preservatives are used in amounts of about 0.1 % to about 1% by weight of the total composition with about 0.1% to about 0.8% being preferred and about 0.1 % to about 0.5% being most preferred.

A cosmetic or pharmaceutical composition according to the invention may also comprise a perfume. Perfumes (fragrance components) and colorants (coloring agents) well known to those skilled in the art may be used in effective amounts to impart the desired fragrance and color to the compositions of the invention.

Furthermore, a cosmetic or pharmaceutical composition of the present invention may also comprise a wax. Suitable waxes which are useful in accord with the invention include: animal waxes, such as beeswax, spermaceti, or wool wax (lanolin); plant waxes, such as carnauba or candelilla; mineral waxes, such as montan wax or ozokerite; and petroleum waxes, such as paraffin wax and microcrystalline wax (a high molecular weight petroleum wax). Animal, plant, and some mineral waxes are primarily esters of a high molecular weight fatty alcohol with a high molecular weight fatty acid. For example, the hexadecanoic acid ester of tricontanol is commonly reported to be a major component of beeswax. Other suitable waxes according to the invention include the synthetic waxes including polyethylene polyoxyethylene and hydrocarbon waxes derived from carbon monoxide and hydrogen.

Representative waxes also include: cerosin; cetyl esters; hydrogenated joioba oil; hydrogenated jojoba wax; hydrogenated rice bran wax; Japan wax; jojoba butter; jojoba oil; jojoba wax; munk wax; montan acid wax; ouricury wax; rice bran wax; shellac wax; sufurized jojoba oil; synthetic beeswax; synthetic jojoba oils; trihydroxystearin; cetyl alcohol; stearyl alcohol; cocoa butter; fatty acids of lanolin; mono-, di- and 25 triglycerides which are solid at 25 DEG C., e.g., glyceyl tribehenate (a triester of behenic acid and glycerine) and C1g-C36 acid triglyceride (a mixture of triesters of C1g-C36 carboxylic acids and glycerine) available from Croda, Inc., New York, N.Y. under the tradenames Syncrowax HRC and Syncrowax HGL-C, respectively; fatty esters which are solid at 25 DEG C.; silicone waxes such as methyloctadecaneoxypolysiloxane and poly (dimethylsiloxy) stearoxysiloxane; stearyl mono- and diethanolamide; rosin and its derivatives such as the abietates of glycol and glycerol; hydrogenated oils solid at 25 DEG C.; and sucroglycerides. Thickeners (viscosity control agents) which may be used in effective amounts in aqueous systems include: algin; carbomers such as carbomer 934, 934P, 940 and 941; cellulose gum; cetearyl alcohol, cocamide DEA, dextrin; gelatin; hydroxyethylcellulose; hydroxypropylcellulose; hydroxypropyl methylcellulose; magnesium aluminum silicate; myristyl alcohol; oat flour; oleamide DEA; oleyl alcohol; PEG-7M; PEG-14M; PEG-9OM; stearamide DEA; stearamide MEA; stearyl alcohol; tragacanth gum; wheat starch; xanthan gum; and the likein the above list of thickeners, DEA is diethanolamine, and MEA is monoethanolamine. Thickeners (viscosity control agents) which may be used in effective amounts in nonaqueous systems include aluminum stearates; beeswax; candelilla wax; carnauba; ceresin; cetearyl alcohol; cetyl alcohol; cholesterol; hydrated silica; hydrogenated castor oil; hydrogenated cottonseed oil; hydrogenated soybean oil; hydrogenated tallow glyceride; hydrogenated vegetable oil; hydroxypropyl cellulose; lanolin alcohol; myristyl alcohol; octytdodecyl stearoyl sulfate; oleyl alcohol; ozokerite; microcystalline wax; paraffin, pentaerythrityl tetraoctanoate; polyacrylamide; polybutene; polyethylene; propylene glycol dicaprylate; propylene glycol dipelargonate; stearalkonium hectorite; stearyl alcohol; stearyl stearate; synthetic beeswax; trihydroxystearin; trilinolein; tristearin; zinc stearate; and the like.

Customary native and synthetic thickeners or gel formers in formulations are crosslinked polyacrylic acids and derivatives thereof, polysaccharides, such as xanthane gum or alginates, carboxymethylcellulose or hydroxycarboxymethylcellulose, hydrocolloids such as gum Arabic or montmorillonite minerals, such as bentonites or fatty alcohols, polyvinyl alcohol and polyvinlypyrrolidone.

Other ingredients which can be added or used in a cosmetic or pharmaceutical composition according to the invention in amounts effective for their intended use, include: biological additives to enhance performance or consumer appeal such as amino acids, proteins, vanilla, aloe extract, bioflavinoids, and the like; buffering agents,; emulsion stabilizers; pH adjusters; opacifying agents; and propellants such as butane carbon dioxide, ethane, hydrochlorofluorocarbons 22 and 142b, hydrofluorocarbon 152a, isobutane, isopentane, nitrogen, nitrous oxide, pentane, propane, and the like.

Furthermore, the preparations according to the invention may also comprise compounds which have an antioxidative, free-radical scavenger, skin moisturizing or moisture-retaining, antierythematous,antiinflammatory or antiallergic action, in order to supplement or enhance their action. In particular, these compounds can be chosen from the group of vitamins, plant extracts, alpha- and beta-hydroxy acids, ceramides, antiinflammatory, antimicrobial or UV-filtering substances, and derivatives thereof and mixtures thereof. Advantageously, preparations according to the invention can also comprise substances which absorb UV radiation in the UV-B and/or UV-A region. The lipid phase is advantageously chosen from the group of substances of mineral oils, mineral waxes, branched and/or unbranched hydrocarbons and hydrocarbon waxes, triglycerides of saturated and/or unsaturated, branched and/or unbranched C.sub.8-C.sub.24-alkanecarboxylic acids; they can be chosen from synthetic, semisynthetic or natural oils, such as olive oil, palm oil, almond oil or mixtures; oils, fats or waxes, esters of saturated and/or unsaturated, branched and/or unbranched C.sub.3-C.sub.30-alkane carboxylic acids and saturated and/or unsaturated, branched and/or unbranched C.sub.3-C.sub.30-alcohols, from aromatic carboxylic acids and saturated and/or unsaturated, branched and/or unbranched C.sub.3-C.sub.30-alcohols, for example isopropyl myristate, isopropyl stearate, hexyldecyl stearate, oleyl oleate; and also synthetic, semisynthetic and natural mixtures of such esters, such as jojoba oil, alkyl benzoates or silicone oils, such as, for example, cyclomethicone, dimethylpolysiloxane, diethylpolysiloxane, octamethylcyclo-tetrasiloxane and mixtures thereof or dialkyl ethers.

The active ingredients according to the invention may, for example, be used in cosmetic compositions for the cleansing of the skin, such as bar soaps, toilet soaps, curd soaps, transparent soaps, luxury soaps, deodorizing soaps, cream soaps, baby soaps, skin protection soaps, abrasive soaps, syndets, liquid soaps, pasty soaps, soft soaps, washing pastes, liquid washing, showering and bath preparations, e.g. washing lotions, shower preparations, shower gels, foam baths, cream foam baths, oil baths, bath extracts, scrub preparations, in-situ products, shaving foams, shaving lotions, shaving creams. In addition, they are suitable for skin cosmetic preparations, such as W/O or O/W skin and body creams, day and night creams, light protection compositions, aftersun products, multiple emulsions, gelees, microemulsions, liposome preparations, niosome preparations, lipogels, sportgels, moisturizing creams, bleaching creams, vitamin creams, skin lotions, care lotions, ampoules, aftershave lotions, preshaves, humectant lotions, cellulite creams, depigmentation compositions, massage preparations, body powders, deodorants, antiperspirants, repellents and others. The term "active ingredient" refers, for example, to the microorganism according to the present invention, mutant, derivative, inactive form, lysate, fraction or extract thereof as described above. Preferably, the term "active ingredient" as used in the compositions herein below is a substitute of, e.g., the microorganisms, mutants, derivatives, inactive forms, lysates, fractions or extracts thereof which are described herein above. If not indicated otherwise, the term "active ingredient" as used in the compositions described below refers to the percentage of, e.g., the microorganism according to the present invention, mutant, derivative, inactive form, lysate, fraction or extract thereof as described above, in the composition. Preferably, the term "active ingredient" refers to *Lactobacillus spec.* as defined herein above, in a concentration of e.g. 10² - 10¹³ cells per ml. More preferably, the term "active ingredient" refers to an solution, e.g. an aqueous solution or any other suitable solution known to the person skilled in the art, comprising up to 0.001 % to up to 99,999% of *Lactobacillus spec.* as defined herein above, in any suitable concentration known to the skilled person, e.g., a concentration of. 10² - 10¹³ cells per ml. Even more preferably, the term refers to a solution comprising up to 0.001%, 0.01%, 0.1%, 1%, 5%, 10%, 15%, 20%, 25%, 30%, 40%, 50%, 60%, 70%, 80%, 90%, 95%, 99%, 99.9%, 99.99% or 99.999%, most preferably comprising up to 0.001 to up to 5%, of *Lactobacillus spec.,* as defined herein above, in any suitable concentration known to the skilled person, e.g. a concentration of. 10² - 10¹³ cells per ml.

In a preferred embodiment, a cosmetic composition comprises a daily care O/W formulation, which may contain, for example, the following ingredients in % in accordance with the International Nomenclature of Cosmetic Ingredients, INCI:

### Active ingredient 1 %:

| | | |
|---|---|---|
| A | 1.7 | ceteareth-6, stearyl alcohol |
| | 0.7 | ceteareth-25 |
| | 2.0 | diethylamino hydroxybenzoyl hexyl benzoate |
| | 2.0 | PEG-14 dimethicone |
| | 3.6 | cetearyl alcohol |
| | 6.0 | ethylhexyl methoxycinnamate |
| | 2.0 | dibutyl adipate |
| B | 5.0 | glycerol |
| | 1.0 | panthenol |
| | q.s. | preservative |
| | 68.6 | aqua dem. |
| C | 4.0 | caprylic/capric triglyceride, sodium acrylates copolymer |
| D | 0.2 | sodium ascorbyl phosphate |
| | 1.0 | tocopheryl acetate |
| | 0.2 | bisabolol |
| | 1.0 | caprylic/capric triglyceride, sodium ascorbate, tocopherol, retinol |
| | 1.0 | active ingredient |
| E | q.s. | sodium hydroxide |

### Active ingredient 5%:

| | | |
|---|---|---|
| A | 1.7 | ceteareth-6, stearyl alcohol |
| | 0.7 | ceteareth-25 |
| | 2.0 | diethylamino hydroxybenzoyl hexyl benzoate |
| | 2.0 | PEG-14 dimethicone |
| | 3.6 | cetearyl alcohol |
| | 6.0 | ethylhexyl methoxycinnamate |
| | 2.0 | dibutyl adipate |
| B | 5.0 | glycerol |
| | 1.0 | panthenol |
| | q.s. | preservative |
| | 64.6 | aqua dem. |
| C | 4.0 | caprylic/capric triglyceride, sodium acrylates copolymer |
| D | 0.2 | sodium ascorbyl phosphate |
| | 1.0 | tocopheryl acetate |
| | 0.2 | bisabolol |
| | 1.0 | caprylic/capric triglyceride, sodium ascorbate, tocopherol, retinol |
| | 5.0 | active ingredient |
| E | q.s. | sodium hydroxide |

Phases A and B are separately heated to app. 80°C. Phase B is subsequently stirred into phase A and homogenized. Phase C is stirred into a combination of phases A and B and homogenized. The mixture is under agitation cooled down to app. 40°C; then phase D is added and the pH is adjusted with phase E to approx. 6.5. The solution is subsequently homogenized and cooled down to room temperature.

In a further preferred embodiment, a cosmetic composition comprises a protecting day cream O/W formulation, which may contain, for example, the following ingredients in % in accordance with the International Nomenclature of Cosmetic Ingredients, INCI:

### Active ingredient 1 %:

| | | |
|---|---|---|
| A | 1.7 | ceteareth-6, stearyl alcohol |
| | 0.7 | ceteareth-25 |
| | 2.0 | diethylamino hydroxybenzoyl hexyl benzoate |
| | 2.0 | PEG-14 dimethicone |
| | 3.6 | cetearyl alcohol |
| | 6.0 | ethylhexyl methoxycinnamate |
| | 2.0 | dibutyl adipate |
| B | 5.0 | glycerol |
| | 1.0 | panthenol |
| | q.s. | preservative |
| | 68.8 | aqua dem. |
| C | 4.0 | caprylic/capric triglyceride, sodium acrylates copolymer |
| D | 1.0 | sodium ascorbyl phosphate |
| | 1.0 | tocopheryl acetate |
| | 0.2 | bisabolol |
| | 1.0 | active ingredient |
| E | q.s. | sodium hydroxide |

### Active ingredient 5%:

| | | |
|---|---|---|
| A | 1.7 | ceteareth-6, stearyl alcohol |
| | 0.7 | ceteareth-25 |
| | 2.0 | diethylamino hydroxybenzoyl hexyl benzoate |
| | 2.0 | PEG-14 dimethicone |
| | 3.6 | cetearyl alcohol |
| | 6.0 | ethylhexyl methoxycinnamate |
| | 2.0 | dibutyl adipate |
| B | 5.0 | glycerol |
| | 1.0 | panthenol |
| | q.s. | preservative |
| | 64.8 | aqua dem. |
| C | 4.0 | caprylic/capric triglyceride, sodium acrylates copolymer |
| D | 1.0 | sodium ascorbyl phosphate |
| | 1.0 | tocopheryl acetate |
| | 0.2 | bisabolol |
| | 5.0 | active ingredient |
| E | | q.s. sodium hydroxide |

Phases A and B are separately heated to app. 80°C. Phase B is subsequently stirred into phase A and homogenized. Phase C is introduced into a combination of phases A and B and homogenized. The mixture is under agitation cooled down to app. 40°C; then phase D is added and the pH is adjusted with phase E to about 6.5. The solution is subsequently homogenized and cooled down to room temperature.

In a further preferred embodiment, a cosmetic composition comprises a skin cleanser O/W formulation, which may contain, for example, the following ingredients in % in accordance with the International Nomenclature of Cosmetic Ingredients, INCI:

### Active ingredient 1%:

| | | |
|---|---|---|
| A | 10.0 | cetearyl ethylhexanoate |
| | 10.0 | caprylic/capric triglyceride |
| | 1.5 | cyclopentasiloxane, cyclohexasilosane |
| | 2.0 | PEG-40 hydrogenated castor oil |
| B | 3.5 | caprylic/capric triglyceride, sodium acrylates copolymer |
| C | 1.0 | tocopheryl acetate |
| | 0.2 | bisabolol |
| | q.s. | preservative |
| | q.s. | perfume oil |
| D | 3.0 | polyquatemium-44 |
| | 0.5 | cocotrimonium methosulfate |
| | 0.5 | ceteareth-25 |
| | 2.0 | panthenol, propylene glycol |
| | 4.0 | propylene glycol |
| | 1.0 | active ingredient |
| | 60.7 | aqua dem. |

### Active ingredient 5%:

| | | |
|---|---|---|
| A | 10.0 | cetearyl ethylhexanoate |
| | 10.0 | caprylic/capric triglyceride |
| | 1.5 | cyclopentasiloxane, cyclohexasilosane |
| | 2.0 | PEG-40 hydrogenated castor oil |
| B | 3.5 | caprylic/capric triglyceride, sodium acrylates copolymer |
| C | 1.0 | tocopheryl acetate |
| | 0.2 | bisabolol |
| | q.s. | preservative |
| | q.s. | perfume oil |
| D | 3.0 | polyquatemium-44 |
| | 0.5 | cocotrimonium methosulfate |
| | 0.5 | ceteareth-25 |
| | 2.0 | panthenol, propylene glycol |
| | 4.0 | propylene glycol |
| | 5.0 | active ingredient |
| | 56.8 | aqua dem. |

Initially, phase A is dissolved and phase B subsequently stirred into phase A. Subsequently, phase C is introduced into the combination of phases A and B. In a next step, phase D is dissolved and stirred into combined phases A, B and C. The mixture is homogenized and stirred for 15 min.

In a further preferred embodiment, a cosmetic composition comprises a daily care body spray formulation, which may contain, for example, the following ingredients in % in accordance with the International Nomenclature of Cosmetic Ingredients, INCI:

### Active ingredient 1 %:

| | | |
|---|---|---|
| A | 3.0 | ethylhexyl methoxycinnamate |
| | 2.0 | diethylamino hydroxybenzoyl hexyl benzoate |
| | 1.0 | polyquatemium-44 |
| | 3.0 | propylene glycol |
| | 2.0 | panthenol, propylene glycol |
| | 1.0 | cyclopentasiloxane, cyclohexasiloxane |
| | 10.0 | octyldodecanol |
| | 0.5 | PVP |
| | 10.0 | caprylic/capric triglyceride |
| | 3.0 | C12-15 alkyl benzoate |
| | 3.0 | glycerol |
| | 1.0 | tocopheryl acetate |
| | 0.3 | bisabolol |
| | 1.0 | active ingredient |
| | 59.2 | alcohol |

### Active ingredient 5%:

| | | |
|---|---|---|
| A | 3.0 | ethylhexyl methoxycinnamate |
| | 2.0 | diethylamino hydroxybenzoyl hexyl benzoate |
| | 1.0 | polyquatemium-44 |
| | 3.0 | propylene glycol |
| | 2.0 | panthenol, propylene glycol |
| | 1.0 | cyclopentasiloxane, cyclohexasiloxane |
| | 10.0 | octyldodecanol |
| | 0.5 | PVP |
| | 10.0 | caprylic/capric triglyceride |
| | 3.0 | C12-15 alkyl benzoate |
| | 3.0 | glycerol |
| | 1.0 | tocopheryl acetate |
| | 0.3 | bisabolol |
| | 5.0 | active ingredient |
| | 55.2 | alcohol |

The components of phase A are weighed out and dissolved until clearness.

In a further preferred embodiment, a cosmetic composition comprises a skin gel, which may contain, for example, the following ingredients in % in accordance with the International Nomenclature of Cosmetic Ingredients, INCI:

### Active ingredient 1 %:

| | | |
|---|---|---|
| | 3.6 | PEG-40 hydrogenated castor oil |
| | 15.0 | alcohol |
| | 0.1 | bisabolol |
| | 0.5 | tocopheryl acetate |
| | q.s. | perfume oil |
| B | 3.0 | panthenol |
| | 0.6 | carbomer |
| | 1.0 | active ingredient |
| | 75.4 | aqua dem, |
| C | 0.8 | triethanolamine |

### Active ingredient 5%:

| | | |
|---|---|---|
| | 3.6 | PEG-40 hydrogenated castor oil |
| | 15.0 | alcohol |
| | 0.1 | bisabolol |
| | 0.5 | tocopheryl acetate |
| | q.s. | perfume oil |
| B | 3.0 | panthenol |
| | 0.6 | carbomer |
| | 5.0 | active ingredient |
| | 71.4 | aqua dem, |
| C | 0.8 | triethanolamine |

Initially, phase A is dissolved until clearness. Phase B is macerated and subsequently neutralized with phase C. In a next step, phase A is stirred into the homogenized phase B and the mixture is homogenized.

In yet a further preferred embodiment, a cosmetic composition comprises an after shave lotion, which may contain, for example, the following ingredients in % in accordance with the International Nomenclature of Cosmetic Ingredients, INCI:

### Active ingredient 1 %:

| | | |
|---|---|---|
| A | 10.0 | cetearyl ethylhexanoate |
| | 5.0 | tocopheryl acetate |
| | 1.0 | bisabolol |
| | 0.1 | perfume oil |
| | 0.3 | acrylates/c10-30 alkyl acrylate crosspolymer |
| B | 15.0 | alcohol |
| | 1.0 | panthenol |
| | 3.0 | glycerol |
| | 1.0 | active ingredient |
| | 0.1 | triethanolamine |
| | 63.5 | aqua dem. |

### Active ingredient 5%:

| | | |
|---|---|---|
| A | 10.0 | cetearyl ethylhexanoate |
| | 5.0 | tocopheryl acetate |
| | 1.0 | bisabolol |
| | 0.1 | perfume oil |
| | 0.3 | acrylates/c10-30 alkyl acrylate crosspolymer |
| B | 15.0 | alcohol |
| | 1.0 | panthenol |
| | 3.0 | glycerol |
| | 5.0 | active ingredient |
| | 0.1 | triethanolamine |
| | 59.5 | aqua dem. |

The component of phase A are mixed. In a next step, phase B is dissolved and introduced into phase A and subsequently homogenized.

In a further preferred embodiment, a cosmetic composition comprises an after sun lotion, which may contain, for example, the following ingredients in % in accordance with the International Nomenclature of Cosmetic Ingredients, INCI:

### Active ingredient 1%:

| | | |
|---|---|---|
| A | 0.4 | acrylates/C10-30 alkyl acrylate crosspolymer |
| | 15.0 | cetearyl ethylhexanoate |
| | 0.2 | bisabolol |
| | 1.0 | tocopheryl acetate |
| | q.s. | perfume oil |
| B | 1.0 | panthenol |
| | 15.0 | alcohol |
| | 3.0 | glycerol |
| | 1.0 | active ingredient |
| | 63.2 | aqua dem, |
| C | 0.2 | triethanolamine |

### Active ingredient 1 %:

| | | |
|---|---|---|
| A | 0.4 | acrylates/C10-30 alkyl acrylate crosspolymer |
| | 15.0 | cetearyl ethylhexanoate |
| | 0.2 | bisabolol |
| | 1.0 | tocopheryl acetate |
| | q.s. | perfume oil |
| B | 1.0 | panthenol |
| | 15.0 | alcohol |
| | 3.0 | glycerol |
| | 5.0 | active ingredient |
| | 59.2 | aqua dem. |
| C | 0.2 | triethanolamine |

The component of phase A are mixed. Phase B introduced into phase A and homogenized. The mixture is neutralized with phase C and subsequently homogenized.

In a further preferred embodiment, a cosmetic composition comprises a body balsam, which may contain, for example, the following ingredients in % in accordance with the International Nomenclature of Cosmetic Ingredients, INCI:

### Active ingredient 1 %:

| | | |
|---|---|---|
| A | 2.0 | ceteareth-6, stearyl alcohol |
| | 2.0 | ceteareth-25 |
| | 5.0 | cetearyl ethylhexanoate |
| | 4.0 | cetyl alcohol |
| | 4.0 | glyceryl stearate |
| | 5.0 | mineral oil |
| | 0.2 | menthol |
| | 0.5 | camphor |
| B | 69.3 | aqua dem. |
| | q.s. | preservative |
| C | 1.0 | bisabolol |
| | 1.0 | tocopheryl acetate |
| D | 1.0 | active ingredient |
| | 5.0 | witch hazel extract |

### Active ingredient 5%:

| | | |
|---|---|---|
| A | 2.0 | ceteareth-6, stearyl alcohol |
| | 2.0 | ceteareth-25 |
| | 5.0 | cetearyl ethylhexanoate |
| | 4.0 | cetyl alcohol |
| | 4.0 | glyceryl stearate |
| | 5.0 | mineral oil |
| | 0.2 | menthol |
| | 0.5 | camphor |
| B | 65.3 | aqua dem. |
| | q.s. | preservative |
| C | 1.0 | bisabolol |
| | 1.0 | tocopheryl acetate |
| D | 5.0 | active ingredient |
| | 5.0 | witch hazel extract |

Phases A and B are separately heated to app. 80°C. Phase B is subsequently stirred into phase A and homogenized. The mixture is under agitation cooled down to app. 40°C; then phases C and D are added. Subsequently, the mixture is homogenized and cooled down to room temperature under agitation.

In a further preferred embodiment, a cosmetic composition comprises a W/O emulsion with bisabolol, which may contain, for example, the following ingredients in % in accordance with the International Nomenclature of Cosmetic Ingredients, INCI:

### Active ingredient 1 %:

| | | |
|---|---|---|
| A | 6.0 | PEG-7 hydrogenated castor oil |
| | 8.0 | cetearyl ethylhexanoate |
| | 5.0 | isopropyl myristate |
| | 15.0 | mineral oil |
| | 0.3 | magnesium stearate |
| | 0.3 | aluminum stearate |
| | 2.0 | PEG-45/dodecyl glycol copolymer |
| B | 5.0 | glycerol |
| | 0.7 | magnesium sulfate |
| | 55.6 | aqua dem. |
| C | 1.0 | active ingredient |
| | 0.5 | tocopheryl acetate |
| | 0.6 | bisabolol |

### Active ingredient 5%:

| | | |
|---|---|---|
| A | 6.0 | PEG-7 hydrogenated castor oil |
| | 8.0 | cetearyl ethylhexanoate |
| | 5.0 | isopropyl myristate |
| | 15.0 | mineral oil |
| | 0.3 | magnesium stearate |
| | 0.3 | aluminum stearate |
| | 2.0 | PEG-45/dodecyl glycol copolymer |
| B | 5.0 | glycerol |
| | 0.7 | magnesium sulfate |
| | 51.6 | aqua dem. |
| C | 5.0 | active ingredient |
| | 0.5 | tocopheryl acetate |
| | 0.6 | bisabolol |

Phases A and B are separately heated to app. 85°C. Phase B is subsequently stirred into phase A and homogenized. The mixture is under agitation cooled down to app. 40°C; then phase C is added. Subsequently, the mixture is shortly homogenized and cooled down to room temperature under agitation.

In a further preferred embodiment, a cosmetic composition comprises a mousse conditioner with holding agent, which may contain, for example, the following ingredients in % in accordance with the International Nomenclature of Cosmetic Ingredients, INCI:

### Active ingredient 1 %:

| | | |
|---|---|---|
| A | 10.0 | PVPNA copolymer |
| | 0.2 | hydroxyethyl cetyldimonium phosphate |
| | 0.2 | ceteareth-25 |
| | 0.5 | dimethicone copolyol |
| | q.s. | perfume oil |
| | 10.0 | alcohol |
| | 1.0 | active ingredient |
| | 68.1 | aqua dem. |
| | 10.0 | propane/butane |

### Active ingredient 5%:

| | | |
|---|---|---|
| A | 10.0 | PVP/VA copolymer |
| | 0.2 | hydroxyethyl cetyldimonium phosphate |
| | 0.2 | ceteareth-25 |
| | 0.5 | dimethicone copolyol |
| | q.s. | perfume oil |
| | 10.0 | alcohol |
| | 5.0 | active ingredient |
| | 64.1 | aqua dem. |
| | 10.0 | propane/butane |

The components of phase A are weighed out and stirred until complete dissolution. Subsequently the mixture is bottled.

In a further preferred embodiment, a cosmetic composition comprises a mousse conditioner, which may contain, for example, the following ingredients in % in accordance with the International Nomenclature of Cosmetic Ingredients, INCI:

### Active ingredient 1%:

| | | |
|---|---|---|
| A | 1.0 | polyquaternium-4 |
| | 0.5 | hydroxyethyl cetyldimonium phosphate |
| | 1.0 | active ingredient |
| | q.s. | perfume oil |
| | q.s. | preservative |
| | 91.5 | aqua dem. |
| | 6.0 | propane/butane |

### Active ingredient 5%:

| | | |
|---|---|---|
| A | 1.0 | polyquatemium-4 |
| | 0.5 | hydroxyethyl cetyldimonium phosphate |
| | 5.0 | active ingredient |
| | q.s. | perfume oil |
| | q.s. | preservative |
| | 87.5 | aqua dem. |
| | 6.0 | propane/butane |

The components of phase A are weighed out and stirred until clear dissolution. Subsequently the mixture is bottled.

In a further preferred embodiment, a cosmetic composition comprises a mousse conditioner, which may contain, for example, the following ingredients in % in accordance with the International Nomenclature of Cosmetic Ingredients, INCI:

### Active ingredient 1 %:

| | | |
|---|---|---|
| A | 1.0 | polyquatemium-11 |
| | 0.5 | hydroxyethyl cetyldimonium phosphate |
| | 1.0 | active ingredient |
| | q.s. | perfume oil |
| | q.s. | preservative |
| | 91.5 | aqua dem. |
| | 6.0 | propane/butane |

### Active ingredient 5%:

| | | |
|---|---|---|
| A | 1.0 | polyquaternium-11 |
| | 0.5 | hydroxyethyl cetyldimonium phosphate |
| | 5.0 | active ingredient |
| | q.s. | perfume oil |
| | q.s. | preservative |
| | 87.5 | aqua dem. |
| | 6.0 | propane/butane |

The components of phase A are weighed out and stirred until clear dissolution. Subsequently the mixture is bottled.

In a further preferred embodiment, a cosmetic composition comprises a styling foam, which may contain, for example, the following ingredients in % in accordance with the International Nomenclature of Cosmetic Ingredients, INCI:

### Active ingredient 1%:

| | | |
|---|---|---|
| A | 0.5 | laureth-4 |
| | q.s. | perfume oil |
| B | 77.3 | aqua dem. |
| | 10.0 | polyquatemium-28 |
| | 1.0 | active ingredient |
| | 0.5 | dimethicone copolyol |
| | 0.2 | ceteareth-25 |
| | 0.2 | panthenol |
| | 0.1 | PEG-25 PABA |
| | 0.2 | hydroxyethylcellulose |
| C | 10.0 | HFC 152 A |

### Active ingredient 5%:

| | | |
|---|---|---|
| A | 0.5 | laureth-4 |
| | q.s. | perfume oil |
| B | 73.3 | aqua dem. |
| | 10.0 | polyquaternium-28 |
| | 5.0 | active ingredient |
| | 0.5 | dimethicone copolyol |
| | 0.2 | ceteareth-25 |
| | 0.2 | panthenol |
| | 0.1 | PEG-25 PABA |
| | 0.2 | hydroxyethylcellulose |
| C | 10.0 | HFC 152 A |

The components of phase A are mixed. Then, the components of phase B are successively added and dissolved. The mixture is bottled with phase C.

In a further preferred embodiment, a cosmetic composition comprises a styling foam, which may contain, for example, the following ingredients in % in accordance with the International Nomenclature of Cosmetic Ingredients, INCI:

### Active ingredient 1%:

| | | |
|---|---|---|
| A | 2.0 | cocotrimonium methosulfate |
| | q.s. | perfume oil |
| B | 78.5 | aqua dem. |
| | 6.7 | acrylates copolymer |
| | 0.6 | AMP |
| | 1.0 | active ingredient |
| | 0.5 | dimethicone copolyol |
| | 0.2 | ceteareth-25 |
| | 0.2 | panthenol |
| | 0.1 | PEG-25 PABA |
| | 0.2 | hydroxyethylcellulose |
| C | 10.0 | HFC 152 A |

### Active ingredient 5%:

| | | |
|---|---|---|
| A | 2.0 | cocotrimonium methosulfate |
| | q.s. | perfume oil |
| B | 74.5 | aqua dem. |
| | 6.7 | acrylates copolymer |
| | 0.6 | AMP |
| | 5.0 | active ingredient |
| | 0.5 | dimethicone copolyol |
| | 0.2 | ceteareth-25 |
| | 0.2 | panthenol |
| | 0.1 | PEG-25 PABA |
| | 0.2 | hydroxyethylcellulose |
| C | 10.0 | HFC 152 A |

The components of phase A are mixed. Then, the components of phase B are successively added and dissolved. The mixture is bottled with phase C.

In a further preferred embodiment, a cosmetic composition comprises a styling foam, which may contain, for example, the following ingredients in % in accordance with the International Nomenclature of Cosmetic Ingredients, INCI:

### Active ingredient 1 %:

| | | |
|---|---|---|
| A | 2.0 | cocotrimonium methosulfate |
| | q.s. | perfume oil |
| B | 7.70 | polyquatemium-44 |
| | 1.0 | active ingredient |
| | q.s. | preservative |
| | 79.3 | aqua dem. |
| C | 10.0 | propane/butane |

### Active ingredient 5%:

| | | |
|---|---|---|
| A | 2.0 | cocotrimonium methosulfate |
| | q.s. | perfume oil |
| B | 7.70 | polyquaternium-44 |
| | 5.0 | active ingredient |
| | q.s. | preservative |
| | 75.3 | aqua dem. |
| C | 10.0 | propane/butane |

The components of phase A are mixed. The components of phase B are dissolved until cloudlessness and subsequently stirred into phase A. The pH is adjusted to 6-7. The mixture is bottled with phase C.

In a further preferred embodiment, a cosmetic composition comprises a styling foam, which may contain, for example, the following ingredients in % in accordance with the International Nomenclature of Cosmetic Ingredients, INCI:

### Active ingredient 1 %:

| | | |
|---|---|---|
| A | 2.00 | cocotrimonium methosulfate |
| | q.s. | perfume oil |
| B | 72.32 | aqua dem. |
| | 2.00 | VP/acrylates/lauryl methacrylate copolymer |
| | 0.53 | AMP |
| | 1.00 | active ingredient |
| | 0.20 | ceteareth-25 |
| | 0.50 | panthenol |
| | 0.05 | benzophenone-4 |
| | 0.20 | amodimethicone, cetrimonium chloride, trideceth-12 |
| | 15.00 | alcohol |
| C | 0.20 | hydroxyethylcellulose |
| D | 6.00 | propane/butane |

### Active ingredient 5%:

| | | |
|---|---|---|
| A | 2.00 | cocotrimonium methosulfate |
| | q.s. | perfume oil |
| B | 68.32 | aqua dem. |
| | 2.00 | VP/acrylates/lauryl methacrylate copolymer |
| | 0.53 | AMP |
| | 5.00 | active ingredient |
| | 0.20 | ceteareth-25 |
| | 0.50 | panthenol |
| | 0.05 | benzophenone-4 |
| | 0.20 | amodimethicone, cetrimonium chloride, trideceth-12 |
| | 15.00 | alcohol |
| C | 0.20 | hydroxyethylcellulose |
| D | 6.00 | propane/butane |

The components of phase A are mixed. The components of phase B are successively added and dissolved. Phase C is dissolved in the mixture of A and B. Subsequently, the pH is adjusted to 6-7 and the mixture is bottled with phase D.

In a further preferred embodiment, a cosmetic composition comprises a styling foam, which may contain, for example, the following ingredients in % in accordance with the International Nomenclature of Cosmetic Ingredients, INCI:

### Active ingredient 1 %:

| | | |
|---|---|---|
| A | 2.00 | cetrimonium chloride |
| | q.s. | perfume oil |
| B | 67.85 | aqua dem. |
| | 7.00 | polyquaternium-46 |
| | 1.00 | active ingredient |
| | 0.20 | ceteareth-25 |
| | 0.50 | panthenol |
| | 0.05 | benzophenone-4 |
| | 0.20 | amodimethicone, cetrimonium chloride, trideceth-12 |
| | 15.00 | alcohol |
| C | 0.20 | hydroxyethylcellulose |
| D | 6.00 | propane/butane |

### Active ingredient 5%:

| | | |
|---|---|---|
| A | 2.00 | cetrimonium chloride |
| | q.s. | perfume oil |
| B | 63.85 | aqua dem. |
| | 7.00 | polyquatemium-46 |
| | 5.00 | active ingredient |
| | 0.20 | ceteareth-25 |
| | 0.50 | panthenol |
| | 0.05 | benzophenone-4 |
| | 0.20 | amodimethicone, cetrimonium chloride, trideceth-12 |
| | 15.00 | alcohol |
| C | 0.20 | hydroxyethylcellulose |
| D | 6.00 | propane/butane |

The components of phase A are mixed. The components of phase B are successively added and dissolved. Phase C is dissolved in the mixture of A and B. Subsequently, the pH is adjusted to 6-7 and the mixture is bottled with phase D.

In a further preferred embodiment, a cosmetic composition comprises a styling foam, which may contain, for example, the following ingredients in % in accordance with the International Nomenclature of Cosmetic Ingredients, INCI:

### Active ingredient 1 %:

| | | |
|---|---|---|
| A | q.s. | PEG-40 hydrogenated castor oil |
| | q.s. | perfume oil |
| | 85.5 | aqua dem. |
| B | 7.0 | sodium polystyrene sulfonate |
| | 1.0 | active ingredient |
| | 0.5 | cetrimonium bromide |
| | q.s. | preservative |
| C | 6.0 | propane/butane |

### Active ingredient 5%:

| | | |
|---|---|---|
| A | q.s. | PEG-40 hydrogenated castor oil |
| | q.s. | perfume oil |
| | 81.5 | aqua dem. |
| B | 7.0 | sodium polystyrene sulfonate |
| | 5.0 | active ingredient |
| | 0.5 | cetrimonium bromide |
| | q.s. | preservative |
| C | 6.0 | propane/butane |

Phase A is solubilized. Then, phase B is weight out into phase A and dissolved until cloudlessness. The pH is adjusted to 6-7 and the mixture is bottled with phase C.

In a further preferred embodiment, a cosmetic composition comprises a styling foam, which may contain, for example, the following ingredients in % in accordance with the International Nomenclature of Cosmetic Ingredients, INCI:

### Active ingredient 1%:

| | | |
|---|---|---|
| A | q.s. | PEG-40 hydrogenated castor oil |
| | q.s. | perfume oil |
| | 92.0 | aqua dem. |
| B | 0.5 | polyquaternium-10 |
| | 1.0 | active ingredient |
| | 0.5 | cetrimonium bromide |
| | q.s. | preservative |
| C | 6.0 | propane/butane |

### Active ingredient 5%:

| | | |
|---|---|---|
| A | q.s. | PEG-40 hydrogenated castor oil |
| | q.s. | perfume oil |
| | 88.0 | aqua dem. |
| B | 0.5 | polyquaternium-10 |
| | 5.0 | active ingredient |
| | 0.5 | cetrimonium bromide |
| | q.s. | preservative |
| C | 6.0 | propane/butane |

Phase A is solubilized. Then, phase B is weight out into phase A and dissolved until cloudlessness. The pH is adjusted to 6-7 and the mixture is bottled with phase C.

In a further preferred embodiment, a cosmetic composition comprises a styling foam, which may contain, for example, the following ingredients in % in accordance with the International Nomenclature of Cosmetic Ingredients, INCI:

### Active ingredient 1%:

| | | |
|---|---|---|
| A | q.s. | PEG-40 hydrogenated castor oil |
| | q.s. | perfume oil |
| | 82.5 | aqua dem. |
| B | 10.0 | polyquaternium-16 |
| | 1.0 | active ingredient |
| | 0.5 | hydroxyethyl cetyldimonium phosphate |
| | q.s. | preservative |
| C | 6.0 | propane/butane |

### Active ingredient 5%:

| | | |
|---|---|---|
| A | q.s. | PEG-40 hydrogenated castor oil |
| | q.s. | perfume oil |
| | 78.5 | aqua dem. |
| B | 10.0 | polyquaternium-16 |
| | 5.0 | active ingredient |
| | 0.5 | hydroxyethyl cetyldimonium phosphate |
| | q.s. | preservative |
| C | 6.0 | propane/butane |

### Phase A is solubilized. Then, phase B is weight out into phase A and dissolved until cloudlessness. The pH is adjusted to 6-7 and the mixture is bottled with phase C.

In a further preferred embodiment, a cosmetic composition comprises a styling foam, which may contain, for example, the following ingredients in % in accordance with the International Nomenclature of Cosmetic Ingredients, INCI:

### Active ingredient 1%:

| | | |
|---|---|---|
| A | 2.0 | cocotrimonium methosulfate |
| | q.s. | perfume oil |
| B | 84.0 | aqua dem. |
| | 2.0 | chitosan |
| | 1.0 | active ingredient |
| | 0.5 | dimethicone copolyol |
| | 0.2 | ceteareth-25 |
| | 0.2 | panthenol |
| | 0.1 | PEG-25 PABA |
| C | 10.0 | HFC 152 A |

### Active ingredient 5%:

| | | |
|---|---|---|
| A | 2.0 | cocotrimonium methosulfate |
| | q.s. | perfume oil |
| B | 80.0 | aqua dem. |
| | 2.0 | chitosan |
| | 5.0 | active ingredient |
| | 0.5 | dimethicone copolyol |
| | 0.2 | ceteareth-25 |
| | 0.2 | panthenol |
| | 0.1 | PEG-25 PABA |
| C | 10.0 | HFC 152 A |

The components of phase A are mixed. The components of phase B are successively added and dissolved. The mixture is bottled with phase C.

In a further preferred embodiment, a cosmetic composition comprises a care shampoo, which may contain, for example, the following ingredients in % in accordance with the International Nomenclature of Cosmetic Ingredients, INCI:

### Active ingredient 1 %:

| | | |
|---|---|---|
| A | 30.0 | sodium laureth sulfate |
| | 6.0 | sodium cocoamphoacetate |
| | 6.0 | cocamidopropyl betaine |
| | 3.0 | sodium laureth sulfate, glycol distearate, cocamide mea, laureth-10 |
| | 1.0 | active ingredient |
| | 7.7 | polyquatemium-44 |
| | 2.0 | amodimethicone |
| | q.s. | perfume oil |
| | q.s. | preservative |
| | 1.0 | sodium chloride |
| | 43.3 | aqua dem. |
| B | q.s. | citric acid |

### Active ingredient 5%:

| | | |
|---|---|---|
| A | 30.0 | sodium laureth sulfate |
| | 6.0 | sodium cocoamphoacetate |
| | 6.0 | cocamidopropyl betaine |
| | 3.0 | sodium laureth sulfate, glycol distearate, cocamide mea, laureth-10 |
| | 5.0 | active ingredient |
| | 7.7 | polyquaternium-44 |
| | 2.0 | amodimethicone |
| | q.s. | perfume oil |
| | q.s. | preservative |
| | 1.0 | sodium chloride |
| | 39.3 | aqua dem. |
| B | q.s. | citric acid |

The components of phase A are mixed and dissolved. The pH is adjusted to 6-7 with phase B, i.e. citric acid.

In a further preferred embodiment, a cosmetic composition comprises a shower gel, which may contain, for example, the following ingredients in % in accordance with the International Nomenclature of Cosmetic Ingredients, INCI:

### Active ingredient 1%:

| | | |
|---|---|---|
| A | 40.0 | sodium laureth sulfate |
| | 5.0 | decyl glucoside |
| | 5.0 | cocamidopropyl betaine |
| | 1.0 | active ingredient |
| | 1.0 | panthenol |
| | q.s. | perfume oil |
| | q.s. | preservative |
| | 2.0 | sodium chloride |
| | 46.0 | aqua dem. |
| B | q.s. | citric acid |

### Active ingredient 5%:

| | | |
|---|---|---|
| A | 40.0 | sodium laureth sulfate |
| | 5.0 | decyl glucoside |
| | 5.0 | cocamidopropyl betaine |
| | 5.0 | active ingredient |
| | 1.0 | panthenol |
| | q.s. | perfume oil |
| | q.s. | preservative |
| | 2.0 | sodium chloride |
| | 42.0 | aqua dem. |
| B | q.s. | citric acid |

The components of phase A are mixed and dissolved. The pH is adjusted to 6-7 with phase B, i.e. citric acid.

In a further preferred embodiment, a cosmetic composition comprises a shampoo, which may contain, for example, the following ingredients in % in accordance with the International Nomenclature of Cosmetic Ingredients, INCI:

### Active ingredient 1%:

| | | |
|---|---|---|
| A | 40.0 | sodium laureth sulfate |
| | 5.0 | sodium C12-15 pareth-15 sulfonate |
| | 5.0 | decyl glucoside |
| | q.s. | perfume oil |
| | 0.1 | phytantriol |
| | 44.6 | aqua dem. |
| | 1.0 | active ingredient |
| | 0.3 | polyquatemium-10 |
| | 1.0 | panthenol |
| | q.s. | preservative |
| | 1.0 | laureth-3 |
| | 2.0 | sodium chloride |

### Active ingredient 5%:

| | | |
|---|---|---|
| A | 40.0 | sodium laureth sulfate |
| | 5.0 | sodium C12-15 pareth-15 sulfonate |
| | 5.0 | decyl glucoside |
| | q.s. | perfume oil |
| | 0.1 | phytantriol |
| | 40.6 | aqua dem. |
| | 5.0 | active ingredient |
| | 0.3 | polyquaternium-10 |
| | 1.0 | panthenol |
| | q.s. | preservative |
| | 1.0 | laureth-3 |
| | 2.0 | sodium chloride |

The components of phase A are mixed and dissolved. The pH is adjusted to 6-7 with citric acid.

In a further preferred embodiment, a cosmetic composition comprises a shampoo ,which may contain, for example, the following ingredients in % in accordance with the International Nomenclature of Cosmetic Ingredients, INCI:

### Active ingredient 1%:

| | | |
|---|---|---|
| A | 15.00 | cocamidopropyl betaine |
| | 10.00 | disodium cocoamphodiacetate |
| | 5.00 | polysorbate 20 |
| | 5.00 | decyl glucoside |
| | q.s. | perfume oil |
| | q.s. | preservative |
| | 1.00 | active ingredient |
| | 0.15 | guar hydroxypropyltrimonium chloride |
| | 2.00 | laureth-3 |
| | 58.00 | aqua dem. |
| | q.s. | citric acid |
| B | 3.00 | PEG-150 distearate |

### Active ingredient 5%:

| | | |
|---|---|---|
| A | 15.00 | cocamidopropyl betaine |
| | 10.00 | disodium cocoamphodiacetate |
| | 5.00 | polysorbate 20 |
| | 5.00 | decyl glucoside |
| | q.s. | perfume oil |
| | q.s. | preservative |
| | 5.00 | active ingredient |
| | 0.15 | guar hydroxypropyltrimonium chloride |
| | 2.00 | laureth-3 |
| | 54.00 | aqua dem. |
| | q.s. | citric acid |
| B | 3.00 | PEG-150 distearate |

The components of phase A are weighed out and dissolved. The pH is adjusted to 6-7. Then, phase B is added and heated up to 50°C. The mixture is cooled down to room temperature under agitation.

In a further preferred embodiment, a cosmetic composition comprises a moistening body care creme, which may contain, for example, the following ingredients in % in accordance with the International Nomenclature of Cosmetic Ingredients, INCI:

### Active ingredient 1%:

| | | |
|---|---|---|
| A | 2.0 | ceteareth-25 |
| | 2.0 | ceteareth-6, stearyl alcohol |
| | 3.0 | cetearyl ethylhexanoate |
| | 1.0 | dimethicone |
| | 4.0 | cetearyl alcohol |
| | 3.0 | glyceryl stearate SE |
| | 5.0 | mineral oil |
| | 4.0 | Simmondsia chinensis (jojoba) seed oil |
| | 3.0 | mineral oil, lanolin alcohol |
| B | 5.0 | propylene glycol |
| | 1.0 | active ingredient |
| | 1.0 | panthenol |
| | 0.5 | magnesium aluminum silicate |
| | q.s | preservative |
| | 65.5 | aqua dem. |
| C | q.s. | perfume oil |
| D | q.s. | citric acid |

### Active ingredient 5%:

| | | |
|---|---|---|
| A | 2.0 | ceteareth-25 |
| | 2.0 | ceteareth-6, stearyl alcohol |
| | 3.0 | cetearyl ethylhexanoate |
| | 1.0 | dimethicone |
| | 4.0 | cetearyl alcohol |
| | 3.0 | glyceryl stearate se |
| | 5.0 | mineral oil |
| | 4.0 | simmondsia chinensis (jojoba) seed oil |
| | 3.0 | mineral oil, lanolin alcohol |
| B | 5.0 | propylene glycol |
| | 5.0 | active ingredient |
| | 1.0 | panthenol |
| | 0.5 | magnesium aluminum silicate |
| | q.s | preservative |
| | 61.5 | aqua dem. |
| C | q.s. | perfume oil |
| D | q.s. | citric acid |

Phases A and B are separately heated to app. 80°C. Phase B is briefly pre-homogenized. Subsequently phase B is stirred into phase A and homogenized. The mixture is cooled down to app. 40°C; then phase C is added. Subsequently, the mixture is well homogenized. The pH is adjusted to 6-7 with phase D, i.e. citric acid.

In a further preferred embodiment, a cosmetic composition comprises a moistening body care creme, which may contain, for example, the following ingredients in % in accordance with the International Nomenclature of Cosmetic Ingredients, INCI:

### Active ingredient 1%:

| | | |
|---|---|---|
| A | 6.0 | PEG-7 hydrogenated castor oil |
| | 10.0 | cetearyl ethylhexanoate |
| | 5.0 | isopropyl myristate |
| | 7.0 | mineral oil |
| | 0.5 | shea butter (butyrospermum parkii) |
| | 0.5 | aluminum stearate |
| | 0.5 | magnesium stearate |
| | 0.2 | bisabolol |
| | 0.7 | quaternium-18-hectorite |
| B | 5.0 | dipropylene glycol |
| | 0.7 | magnesium sulfate |
| | q.s. | preservative |
| | 62.9 | aqua dem. |
| C | q.s. | perfume oil |
| | 1.0 | active ingredient |

### Active ingredient 5%:

| | | |
|---|---|---|
| A | 6.0 | PEG-7 hydrogenated castor oil |
| | 10.0 | cetearyl ethylhexanoate |
| | 5.0 | isopropyl myristate |
| | 7.0 | mineral oil |
| | 0.5 | shea butter (butyrospermum parkii) |
| | 0.5 | aluminum stearate |
| | 0.5 | magnesium stearate |
| | 0.2 | bisabolol |
| | 0.7 | quaternium-18-hectorite |
| B | 5.0 | dipropylene glycol |
| | 0.7 | magnesium sulfate |
| | q.s. | preservative |
| | 58.9 | aqua dem. |
| C | q.s. | perfume oil |
| | 5.0 | active ingredient |

Phases A and B are separately heated to app. 80°C. Phase B is stirred into phase A and homogenized. The mixture is cooled down under agitation to app. 40°C; then phase C is added. Subsequently, the mixture is homogenized. The mixture is cooled down to room temperature under agitation.

In a further preferred embodiment, a cosmetic composition comprises an antitranspiration roll-on, which may contain, for example, the following ingredients in % in accordance with the International Nomenclature of Cosmetic Ingredients, INCI:

### Active ingredient 1 %:

| | | |
|---|---|---|
| A | 0.40 | hydroxyethylcellulose |
| | 50.0 | aqua dem. |
| B | 25.0 | alcohol |
| | 0.1 | bisabolol |
| | 0.3 | farnesol |
| | 2.0 | PEG-40 hydrogenated castor oil q.s. perfume oil |
| C | 3.0 | dipropylene glycol |
| | 3.0 | PEG-14 demethicone |
| | 3.0 | polyquaternium-16 |
| | 8.2 | aqua dem. |
| D | 1.0 | active ingredient |

### Active ingredient 5%:

| | | |
|---|---|---|
| A | 0.40 | hydroxyethylcellulose |
| | 46.0 | aqua dem. |
| B | 25.0 | alcohol |
| | 0.1 | bisabolol |
| | 0.3 | farnesol |
| | 2.0 | PEG-40 hydrogenated castor oil q.s. perfume oil |
| C | 3.0 | dipropylene glycol |
| | 3.0 | PEG-14 demethicone |
| | 3.0 | polyquaternium-16 |
| | 8.2 | aqua dem. |
| D | 5.0 | active ingredient |

Phase A is swollen, phases B and C are solubilized independently. Subsequently, phases B and A are stirred into phase C. Finally, phase D is added.

In a further preferred embodiment, a cosmetic composition comprises a transparent deo stick, which may contain, for example, the following ingredients in % in accordance with the International Nomenclature of Cosmetic Ingredients, INCI:

### Active ingredient 1%:

| | | |
|---|---|---|
| A | 3.0 | ceteareth-25 |
| | 3.0 | PEG-40 hydrogenated castor oil |
| | 0.2 | bisabolol rac. |
| | 1.0 | tocopheryl acetate |
| | 3.0 | perfume oil |
| | 5.0 | sodium stearate |
| | 15.0 | glycerol 87% |
| | 60.0 | propylene glycol |
| | 9.3 | aqua dem. |
| B | 1.0 | active ingredient |

### Active ingredient 5%:

| | | |
|---|---|---|
| A | 3.0 | ceteareth-25 |
| | 3.0 | PEG-40 hydrogenated castor oil |
| | 0.2 | bisabolol rac. |
| | 1.0 | tocopheryl acetate |
| | 3.0 | perfume oil |
| | 5.0 | sodium stearate |
| | 15.0 | glycerol 87% |
| | 60.0 | propylene glycol |
| | 5.3 | aqua dem. |
| B | 5.0 | active ingredient |

Components of phase A are weighed out and melted. Subsequently, phase B is added.

In a further preferred embodiment, a cosmetic composition comprises an antitranspiration spray, which may contain, for example, the following ingredients in % in accordance with the International Nomenclature of Cosmetic Ingredients, INCI:

### Active ingredient 1%:

| | | |
|---|---|---|
| A | 3.0 | PEG-40 hydrogenated castor oil |
| | 0.2 | phytantriol |
| | 0.5 | perfume oil |
| | 40.0 | alcohol |
| B | 53.49 | aqua dem. |
| | 2.0 | propylene glycol |
| | 0.5 | panthenol |
| | 0.01 | BHT |
| C | 1.0 | active ingredient |

### Active ingredient 5%:

| | | |
|---|---|---|
| A | 3.0 | PEG-40 hydrogenated castor oil |
| | 0.2 | phytantriol |
| | 0.5 | perfume oil |
| | 40.0 | alcohol |
| B | 49.49 | aqua dem. |
| | 2.0 | propylene glycol |
| | 0.5 | panthenol |
| | 0.01 | BHT |
| C | 5.0 | active ingredient |

Phase A is solubilized. In a next step the components of phase B added successively. Finally, phase C is added.

In a further preferred embodiment, a cosmetic composition comprises a deo-stick, which may contain, for example, the following ingredients in % in accordance with the International Nomenclature of Cosmetic Ingredients, INCI:

### Active ingredient 1 %:

| | | |
|---|---|---|
| A | 26.0 | stearyl alcohol |
| | 60.0 | cyclopentasiloxane, cyclohexasiloxane |
| | 5.0 | PEG-40 hydrogenated castor oil |
| | 2.5 | isopropyl palmitate |
| B | 1.44 | perfume oil |
| | 0.05 | BHT |
| C | 1.0 | active ingredient |

### Active ingredient 5%:

| | | |
|---|---|---|
| A | 26.0 | stearyl alcohol |
| | 56.0 | cyclopentasiloxane, cyclohexasiloxane |
| | 5.0 | PEG-40 hydrogenated castor oil |
| | 2.5 | isopropyl palmitate |
| B | 1.44 | perfume oil |
| | 0.05 | BHT |
| C | 5.0 | active ingredient |

The components of phase A are weighed out and melted. Phase A is subsequently cooled down while stirring to about 50°C. The components of phase B and C are homogenized and added successively.

In a further preferred embodiment, a cosmetic composition comprises a transparent deo-roll on, which may contain, for example, the following ingredients in % in accordance with the International Nomenclature of Cosmetic Ingredients, INCI:

### Active ingredient 1 %:

| | | |
|---|---|---|
| A | 0.40 | hydroxyethylcellulose |
| | 50.0 | aqua dem. |
| B | 2.0 | PEG-40 hydrogenated castor oil |
| | 0.1 | bisabolol |
| | 0.3 | farnesol |
| | 0.5 | perfume oil |
| | 7.6 | aqua dem. |
| | 25.0 | alcohol |
| C | 3.0 | propylene glycol |
| | 3.0 | PEG-14 demethicone |
| | 3.0 | polyquaternium-16 |
| | 0.1 | allantoin |
| D | 1.0 | active ingredient |

### Active ingredient 5%:

| | | |
|---|---|---|
| A | 0.40 | hydroxyethylcellulose |
| | 46.0 | aqua dem. |
| B | 2.0 | PEG-40 hydrogenated castor oil |
| | 0.1 | bisabolol |
| | 0.3 | farnesol |
| | 0.5 | perfume oil |
| | 7.6 | aqua dem. |
| | 25.0 | alcohol |
| C | 3.0 | propylene glycol |
| | 3.0 | PEG-14 demethicone |
| | 3.0 | polyquaternium-16 |
| | 0.1 | allantoin |
| D | 5.0 | active ingredient |

Phase A is swollen, phase B is solubilized. Subsequently, phase C is added and stirred. Finally, phases B, C and D are stirred into phase A.

In a further preferred embodiment, a cosmetic composition comprises an emulsion, which may contain, for example, the following ingredients in % in accordance with the International Nomenclature of Cosmetic Ingredients, INCI:

### Active ingredient 1%:

| | | |
|---|---|---|
| A | 1.5 | ceteareth-6, stearyl alcohol |
| | 2.0 | ceteareth-25 |
| | 5.0 | PEG-40 hydrogenated castor oil |
| | 1.5 | glyceryl stearate |
| | 1.0 | cetearyl alcohol |
| | 0.5 | Eucerinum anhydricum |
| | 0.2 | phytantriol |
| | 1.0 | cetyl palpitate |
| | 5.0 | dicaprylyl ether |
| | 0.3 | farnesol |
| B | q.s. | preservative |
| | 72.0 | aqua dem. |
| C | q.s. | perfume oil |
| D | 1.0 | active ingredient: |

### Active ingredient 5%:

| | | |
|---|---|---|
| A | 1.5 | ceteareth-6, stearyl alcohol |
| | 2.0 | ceteareth-25 |
| | 5.0 | PEG-40 hydrogenated castor oil |
| | 1.5 | glyceryl stearate |
| | 1.0 | cetearyl alcohol |
| | 0.5 | Eucerinum anhydricum |
| | 0.2 | phytantriol |
| | 1.0 | cetyl palpitate |
| | 5.0 | dicaprylyl ether |
| | 0.3 | farnesol |
| B | q.s. | preservative |
| | 68.0 | aqua dem. |
| C | q.s. | perfume oil |
| D | 5.0 | active ingredient: |

Phases A and B are heated separtely to approx. 80°C. Phase B is stirred into phase A and homogenized for 3 minutes. Subsequently, the mixture is cooled down to 40°C and phases C and D are added. Finally, the mixture is stirred and cooled down to room temperature.

In a further preferred embodiment, a cosmetic composition comprises a deo-pump spray, which may contain, for example, the following ingredients in % in accordance with the International Nomenclature of Cosmetic Ingredients, INCI:

### Active ingredient 1%:

| | | |
|---|---|---|
| A | 5.0 | PEG-40 hydrogenated castor oil |
| | 0.3 | PEG-7 hydrogenated castor oil |
| | 1.0 | glyceryl stearate |
| | 1.0 | cetearyl alcohol |
| | 5.0 | cyclopentasiloxane |
| | 0.5 | Eucerinum anhydricum |
| | 0.2 | phytantriol |
| | 5.0 | dicaprylyl ether |
| | 0.3 | farnesol |
| B | q.s. | preservative |
| | 76.7 | aqua dem. |
| C | q.s. | perfume oil |
| D | 1.0 | active ingredient |

### Active ingredient 5%:

| | | |
|---|---|---|
| A | 5.0 | PEG-40 hydrogenated castor oil |
| | 0.3 | PEG-7 hydrogenated castor oil |
| | 1.0 | glyceryl stearate |
| | 1.0 | cetearyl alcohol |
| | 6.0 | cyclopentasiloxane |
| | 0.5 | Eucerinum anhydricum |
| | 0.2 | phytantriol |
| | 5.0 | dicaprylyl ether |
| | 0.3 | farnesol |
| B | q.s. | preservative |
| | 72.7 | aqua dem. |
| C | q.s. | perfume oil |
| D | 5.0 | active ingredient |

Phases A and B are heated separately to approx. 80°C. Phase B is homogenized and stirred into phases A and C. Subsequently, the mixture is cooled down to 40°C and phase D is added. Finally, the mixture is stirred and cooled down to room temperature.

In a further preferred embodiment, a cosmetic composition comprises a deo-lotion, which may contain, for example, the following ingredients in % in accordance with the International Nomenclature of Cosmetic Ingredients, INCI:

### Active ingredient 1 %:

| | | |
|---|---|---|
| A | 1.5 | ceteareth-6, stearyl alcohol |
| | 1.5 | ceteareth-25) |
| | 2.0 | PEG-40 hydrogenated castor oil |
| | 2.0 | glyceryl stearate |
| | 2.0 | cetearyl alcohol |
| | 2.0 | cetyl alcohol |
| | 2.0 | hydrogenated coco-glycerides |
| | 8.0 | decyl oleate |
| | 0.5 | PEG-14 demehicone |
| | 0.3 | farnesol |
| B | q.s. | preservative |
| | 75.2 | aqua dem. |
| C | q.s. | perfume oil |
| D | 1.0 | active ingredient 1%: |

### Active ingredient 5%:

| | | |
|---|---|---|
| A | 1.5 | ceteareth-6, stearyl alcohol |
| | 1.5 | ceteareth-25 |
| | 2.0 | PEG-40 hydrogenated castor oil |
| | 2.0 | glyceryl stearate |
| | 2.0 | cetearyl alcohol |
| | 2.0 | cetyl alcohol |
| | 2.0 | hydrogenated coco-glycerides |
| | 8.0 | decyl oleate |
| | 0.5 | PEG-14 demehicone |
| | 0.3 | farnesol |
| B | q.s. | preservative |
| | 71.2 | aqua dem. |
| C | q.s. | perfume oil |
| D | 5.0 | active ingredient 1 %: |

Phases A and B are heated separately to approx. 80°C. Phase B is homogenized and stirred into phase A. Subsequently, the mixture is cooled down to 40°C and phases C and D are added. Finally, the mixture is stirred and cooled down to room temperature.

In a further preferred embodiment, a cosmetic composition comprises a deo-lotion, type O/W which may contain, for example, the following ingredients in % in accordance with the International Nomenclature of Cosmetic Ingredients, INCI:

### Active ingredient 1%:

| | | |
|---|---|---|
| A | 2.0 | ceteareth-6, stearyl alcohol |
| | 2.0 | ceteareth-25 |
| | 4.0 | cetearyl ethylhexanoate |
| | 2.0 | cetearyl alcohol |
| | 2.0 | hydrogenated coco-glycerides |
| | 1.0 | glyceryl stearate |
| | 1.0 | mineral oil |
| | 0.5 | dimethicone |
| | 0.2 | bisabolol |
| B | 2.0 | panthenol, propylene glycol |
| | 2.0 | propylene glycol |
| | q.s. | preservative |
| | 79.8 | aqua dem. |
| C | 1.2 | caprylic/capric triglyceride, sodium acrylates copolymer |
| D | 0.2 | tocopherol |
| | q.s. | perfume oil |
| E | 1.0 | active ingredient: |

### Active ingredient 5%:

| | | |
|---|---|---|
| A | 2.0 | ceteareth-6, stearyl alcohol |
| | 2.0 | ceteareth-25 |
| | 4.0 | cetearyl ethylhexanoate |
| | 2.0 | cetearyl alcohol |
| | 2.0 | hydrogenated coco-glycerides |
| | 1.0 | glyceryl stearate |
| | 1.0 | mineral oil |
| | 0.5 | dimethicone |
| | 0.2 | bisabolol |
| B | 2.0 | panthenol, propylene glycol |
| | 2.0 | propylene glycol |
| | q.s. | preservative |
| | 75.8 | aqua dem. |
| C | 1.2 | caprylic/capric triglyceride, sodium acrylates copolymer |
| D | 0.2 | tocopherol |
| | q.s. | perfume oil |
| E | 5.0 | active ingredient: |

Phases A and B are heated separately to approx. 80°C. Subsequently, phase C is stirred into phases A and B and homogenized. Finally, the mixture is cooled down to 40°C and phases D and E are added.

In a further preferred embodiment, a cosmetic composition comprises a clear shampoo, which may contain, for example, the following ingredients in % in accordance with the International Nomenclature of Cosmetic Ingredients, INCI:

| **Ingredients (in %)** | **Example 1** | **Example 2** | **Example 3** | **Example 4** | **Example 5** |
|---|---|---|---|---|---|
| sodium laureth sulfate | 13.00 | 15.00 | 10.50 | 12.50 | 10.00 |
| codamidopropyl betaine | 7.50 | 7.00 | 5.00 | 5.50 | 10.00 |
| PEG-7 glyceryl cocoate | 2.00 | 2.50 | 3.50 | 5.00 | 2.30 |
| perfume oil | 0.10 | 0.10 | 0.10 | 0.10 | 0.10 |
| active ingredient | 1.0 | 5.0 | 0.1 | 0.5 | 10.0 |
| D-panthenol USP | 1.00 | 1.50 | 1.80 | 1.70 | 1.40 |
| preservative | 0.10 | 0.10 | 0.10 | 0.10 | 0.10 |
| citric acid | 0.10 | 0.10 | 0.10 | 0.10 | 0.10 |
| luviquat ultra care | 1.50 | 1.00 | 1.50 | 1.20 | 1.10 |
| sodium chloride | 1.50 | 1.40 | 1.40 | 1.30 | 1.50 |
| aqua dem. | ad 100 | ad 100 | ad 100 | ad 100 | ad 100 |

**In** a further preferred embodiment, a cosmetic composition comprises a shampoo, which may contain, for example, the following ingredients in % in accordance with the International Nomenclature of Cosmetic Ingredients, INCI:

| **Ingredients (in %)** | **Example 1** | **Example 2** | **Example 3** | **Example 4** | **Example 5** |
|---|---|---|---|---|---|
| sodium laureth sulfate | 35.00 | 40.00 | 30.00 | 45.00 | 27.00 |
| decyl glucoside | 5.00 | 5.50 | 4.90 | 3.50 | 7.00 |
| cocamidopropyl betaine | 10.00 | 5.00 | 12.50 | 7.50 | 15.00 |
| perfume oil | 0.10 | 0.10 | 0.10 | 0.10 | 0.10 |
| active ingredient | 1.0 | 5.0 | 0.1 | 0.5 | 10.0 |
| d-panthenol usp | 0.50 | 1.00 | 0.80 | 1.50 | 0.50 |
| preservative | 0.10 | 0.10 | 0.10 | 0.10 | 0.10 |
| citric acid | 0.10 | 0.10 | 0.10 | 0.10 | 0.10 |
| laureth-3 | 0.50 | 2.00 | 0.50 | 0.50 | 2.00 |
| sodium chloride | 1.50 | 1.50 | 1.50 | 1.50 | 1.50 |
| aqua dem. | ad 100 | ad 100 | ad 100 | ad 100 | ad 100 |

In a further preferred embodiment, a cosmetic composition comprises a clear conditioning shampoo, which may contain, for example, the following ingredients in % in accordance with the International Nomenclature of Cosmetic Ingredients, INCI:

| **Ingredients (in %)** | **Example 1** | **Example 2** | **Example 3** | **Example 4** | **Example 5** |
|---|---|---|---|---|---|
| disodium cocoamphodiacetate | 10.00 | 15.00 | 20.00 | 12.00 | 17.00 |
| decyl glucoside | 5.00 | 6.00 | 7.00 | 8.00 | 4.00 |
| cocamidopropyl betaine | 15.00 | 12.00 | 10.00 | 18.00 | 20.00 |
| Luviquat FC 550 | 0.30 | 0.20 | 0.20 | 0.20 | 0.30 |
| perfume oil | 0.10 | 0.10 | 0.10 | 0.10 | 0.10 |
| active ingredient | 20.0 | 5.0 | 1.0 | 0.5 | 10.0 |
| cremophor PS 20 | 5.00 | 1.00 | 1.00 | 7.00 | 5.00 |
| preservativee | 0.10 | 0.10 | 0.10 | 0.10 | 0.10 |
| laureth-3 | 2.00 | 1.00 | 0.50 | 2.00 | 2.00 |
| citric acid | 0.20 | 0.20 | 0.20 | 0.20 | 0.20 |
| PEG-12 distearate | 3.00 | 2.00 | 2.00 | 3.00 | 2.50 |
| aqua dem. | ad 100 | ad 100 | ad 100 | ad 100 | ad 100 |

In a further preferred embodiment, a cosmetic composition comprises a foam O/W emulsions, which may contain, for example, the following ingredients in % in accordance with the International Nomenclature of Cosmetic Ingredients, INCI:

| **Ingredients (in %)** | **Example1** | | **Example 2** | |
|---|---|---|---|---|
| | W.-% | Vol-% | W.-% | Vol-% |
| stearic acid | 5.00 | | 1.00 | |
| cetyl alcohol | 5.50 | | | |
| cetearyl alcohol | | | 2.00 | |
| PEG-40 stearate | 8.50 | | | |
| PEG-20 stearate | | | 1.00 | |
| caprylic/capric triglyceride | 4.00 | | 2.00 | |
| C12-15 alkyl benzoate | 10.00 | | 15.00 | |
| cyclomethicone | 4.00 | | | |
| dimethicone | | | 0.50 | |
| active ingredient | 5.0 | | 10.0 | |
| ethylhexyl isostearate | | | 5.00 | |
| myristyl myristate | | | 2.00 | |
| ceresin | 1.50 | | | |
| glycerol | | | 3.00 | |
| hydroxypropyl starch phosphate | 1.00 | | 3.50 | |
| BHT | | | 0.02 | |
| perfume oil, preservative | q.s. | | q.s. | |
| colorant | q.s. | | q.s. | |
| potassium hydroxide | q.s. | | q.s. | |
| aqua dem. | ad 100 | | ad 100 | |

| | pH adjusted to 6.5-7.5 | | pH adjusted to 5.0-6.0 | |
|---|---|---|---|---|
| emulsion 1 | | 70 | | |
| emulsion 2 | | | | 35 |
| nitrogen | | 30 | | |
| propan/butan | | | | 65 |

In a further preferred embodiment, a cosmetic composition comprises a conditioning shampoo with pearl brilliance, which may contain, for example, the following ingredients in % in accordance with the International Nomenclature of Cosmetic Ingredients, INCI:

| **Ingredients (in %)** | **Example 1** | **Example 2** | **Example 3** |
|---|---|---|---|
| polyquatemium-10 | 0.50 | 0.50 | 0.40 |
| sodium laureth sulfate | 9.00 | 8.50 | 8.90 |
| codamidopropyl betaine | 2.50 | 2.60 | 3.00 |
| Uvinul® MS 40 | 1.50 | 0.50 | 1.00 |
| active ingredient | 1.0 | 5.0 | 0.5 |
| pearl brilliance solution | 2.00 | 2.50 | |
| preservative, perfume oil, thickener | q.s. | q.s. | q.s. |
| aqua dem. | ad 100 | ad 100 | ad 100 |
| pH adjusted to 6.0 | | | |

In a further preferred embodiment, a cosmetic composition comprises a clear conditioning shampoo, which may contain, for example, the following ingredients in % in accordance with the International Nomenclature of Cosmetic Ingredients, INCI:

| **Ingredients (in %)** | **Example 1** | **Example 2** | **Example 3** |
|---|---|---|---|
| polyquatemium-10 | 0.50 | 0.50 | 0.50 |
| sodium laureth sulfate | 9.00 | 8.50 | 9.50 |
| active ingredient | 5.0 | 0.1 | 3.0 |
| Uvinul M® 40 | 1.00 | 1.50 | 0.50 |
| | 0.20 | 0.20 | 0.80 |
| preservative, perfume oil, thickener | q.s. | q.s. | q.s. |
| aqua dem. | ad 100 | ad 100 | ad 100 |
| pH adjusted to 6.0 | | | |

In a further preferred embodiment, a cosmetic composition comprises a clear conditioner shampoo with volume effect, which may contain, for example, the following ingredients in % in accordance with the International Nomenclature of Cosmetic Ingredients, INCI:

| **Ingredients (in %)** | **Example 1** | **Example 2** | **Example 3** |
|---|---|---|---|
| sodium laureth sulfate | 10.00 | 10.50 | 11.00 |
| Uvinul® MC 80 | 2.00 | 1.50 | 2.30 |
| active ingredient | 10.0 | 0.1 | 0.5 |
| cocamidopropyl betaine | 2.50 | 2.60 | 2.20 |
| preservative, perfume oil, thickener | q.s. | q.s. | q.s. |
| aqua dem. | ad 100 | ad 100 | ad 100 |
| pH adjusted to 6.0 | | | |

In a further preferred embodiment, a cosmetic composition comprises a gel creme, which may contain, for example, the following ingredients in % in accordance with the International Nomenclature of Cosmetic Ingredients, INCI:

| **Ingredients (in %)** | **Example 1** | **Example 2** | **Example 3** | **Example 4** |
|---|---|---|---|---|
| acrylates/C10-30 alkylacrylate crosspolymer | 0.40 | 0.35 | 0.40 | 0.35 |
| carbomer | 0.20 | 0.22 | 0.20 | 0.22 |
| xanthan gum | 0.10 | 0.13 | 0.10 | 0.13 |
| cetearyl alcohol | 3.00 | 2.50 | 3.00 | 2.50 |
| C12-15 alkyl benzoate | 4.00 | 4.50 | 4.00 | 4.50 |
| caprylic/capric triglyceride | 3.00 | 3.50 | 3.00 | 3.50 |
| Uvinul® A Plus^{™} | 2.00 | 1.50 | 0.75 | 1.00 |
| UvaSorb® k2A Ethylhexyl Bis-Isopentylbenzoxazolylphenyl Melamine | | 3.00 | | |
| Uvinul® MC 80 | 3.00 | | 1.00 | |
| bis-ethylhexyloxyphenol methoxyphenyl triazine | | 1.50 | | 2.00 |
| butyl methoxydibenzoylmethane | | | 2.00 | |
| disodium phenyl dibenzimidazole tetrasulfonate | 2.50 | | 0.50 | 2.00 |
| Uvinul® T 150 | 4.00 | | 3.00 | 4.00 |
| octocrylene | | 4.00 | | |
| diethylhexyl butamido triazone | 1.00 | | | 2.00 |
| phenylbenzimidazole sulfonic acid | 0.50 | | 3.00 | |
| methylene bis-benzotriazolyl tetramethylbutylphenol | 2.00 | | 0.50 | 1.50 |
| ethylhexyl salicylate | | | 3.00 | |
| drometrizole trisiloxane | | | 0.50 | |
| terephthaliden dicamphor sulfonic acid | | 1.50 | | 1.00 |
| diethylhexyl 2,6-naphthalate | 3,50 | 4,00 | 7,00 | 9,00 |
| titanium dioxide- microfine | 1.00 | | 3.00 | |
| zinc oxide- microfine | | | | 0.25 |
| active ingredient | 0.1 | 0.5 | 1.0 | 0.02 |
| cyclomethicone | 5.00 | 5.50 | 5.00 | 5.50 |
| dimethicone | 1.00 | 0.60 | 1.00 | 0.60 |
| glycerol | 1.00 | 1.20 | 1.00 | 1.20 |
| sodium hydroxide | q.s. | q.s. | q.s. | q.s. |
| preservative | 0.30 | 0.23 | 0.30 | 0.23 |
| perfume oil | 0.20 | | 0.20 | |
| aqua dem. | ad 100 | ad 100 | ad 100 | ad 100 |
| pH adjusted to 6.0 | | | | |

In a further preferred embodiment, a cosmetic composition comprises a hydrodispersion, which may contain, for example, the following ingredients in % in accordance with the International Nomenclature of Cosmetic Ingredients, INCI:

| **Ingredients (in %)** | **Example 1** | **Example 2** | **Example 3** | **Example 4** | **Example 5** |
|---|---|---|---|---|---|
| ceteaereth-20 | 1.00 | | | 0.50 | |
| cetyl alcohol | | | 1.00 | | |
| sodium carbomer | | 0.20 | | 0.30 | |
| acrylates/C10-30 alkyl acrylate crosspolymer | 0.50 | | 0.40 | 0.10 | 0.50 |
| xanthan gum | | 0.30 | 0.15 | | |
| active ingredient | 5.0 | 0.5 | 3.0 | 0.1 | 10.0 |
| Uvinul® A Plus^{™} | 2.00 | 1.50 | 0.75 | 1.00 | 2.10 |
| UvaSorb® k2A ethylhexyl bis-isopentylbenzoxazolylphenyl melamine | | 3.50 | | | |
| ethylhexyl methoxycinnamate Uvinul® MC 80 | | | | | 5.00 |
| bis-ethylhexyloxyphenol methoxyphenyl triazine | | 1.50 | | 2.00 | 2.50 |
| butylmethoxy dibenzoylmethane | | | 2.00 | | 2.00 |
| dinatrium phenyl dibenzimidazole tetrasulfonate | 2.50 | | 0.50 | 2.00 | |
| ethyhexyl triazone Uvinul® T 150 | 4.00 | | 3.00 | 4.00 | |
| octocrylene | | 4.00 | | | |
| diethylhexyl butamido triazone | 1.00 | | | 2.00 | 1.00 |
| phenylbenzimidazol sulfonic acid | 0.50 | | 3.00 | | |
| methylene bis-benzotriazolyl tetramethylbutylphenol | 2.00 | | 0.50 | 1.50 | 2.50 |
| ethylhexyl salicylate | | | 3.00 | | |
| drometrizol trisiloxane | | | 0.50 | | |
| terephthaliden dicamphor sulfonic acid | | 1.50 | | 1.00 | 1.00 |
| diethylhexyl 2,6-naphthalate | | | 7.00 | | 9.00 |
| titanium dioxide-microfine | 1.00 | | 3.00 | | 3.50 |
| zinc oxide- microfine | | | | 0.25 | |
| C12-15 alkyl benzoate | 2.00 | 2.50 | | | |
| dicapryl ether | | 4.00 | | | |
| butylenglycol dicaprylate/dicaprate | 4.00 | | 2.00 | 6.00 | |
| dicapryl carbonate | | 2.00 | 6.00 | | |
| dimethicone | | 0.50 | 1.00 | | |
| phenyl trimethicone | 2.00 | | 0.50 | | |
| butyrospermum parkii | | 2.00 | | 5.00 | |
| (shea butter) | | | | | |
| VP/hexadecene copolymer | 0.50 | | | 0.50 | 1.00 |
| tricontanyl PVP | 0.50 | | 1.00 | | |
| ethylhexylglycerol | | | 1.00 | | 0.80 |
| glycerol | 3.00 | 7.50 | | 7.50 | 8.50 |
| glycine soja (soybean) oil | | | 1.50 | | 1.00 |
| vitamin E acetate | 0.50 | | 0.25 | | 1.00 |
| glucosylrutin | 0.60 | | | 0.25 | |
| biosaccaride gum-1 | | 2.50 | 0.50 | | 2.00 |
| DMDM hydantoin | | 0.60 | 0.45 | 0.25 | |
| iodopropynyl butylcarbamatec | 0.20 | | | | |
| methylparaben | 0.50 | | 0.25 | 0.15 | |
| phenoxyethanol | 0.50 | 0.40 | | 1.00 | |
| ethanol | 3.00 | 2.00 | 1.50 | | 7.00 |
| perfume oil | 0.20 | | 0.05 | 0.40 | |
| aqua dem. | ad 100 | ad 100 | ad 100 | ad 100 | ad 100 |

In a further preferred embodiment, a cosmetic composition comprises a stick, which may contain, for example, the following ingredients in % in accordance with the International Nomenclature of Cosmetic Ingredients, INCI:

| **Ingredients (in %)** | **Example 1** | **Example 2** | **Example 3** | **Example 4** |
|---|---|---|---|---|
| caprylic/capric triglyceride | 12.00 | 10.00 | 6.00 | |
| octyldodecanol | 7.00 | 14.00 | 8.00 | 3.00 |
| butylene glycol dicaprylate/dicaprate | | | | 12.00 |
| pentaerythrityl | 10.00 | 6.00 | 8.00 | 7.00 |
| tetraisostearate | | | | |
| polyglyceryl-3 diisostearate | 2.50 | | | |
| bis-diglyceryl polyacyladipate-2 | 9.00 | 8.00 | 10.00 | 8.00 |
| cetearyl alcohol | 8.00 | 11.00 | 9.00 | 7.00 |
| myristyl myristate | 3.50 | 3.00 | 4.00 | 3.00 |
| beeswax | 5.00 | 5.00 | 6.00 | 6.00 |
| copernicia cerifera(camauba) wax | 1.50 | 2.00 | 2.00 | 1.50 |
| cera alba | 0.50 | 0.50 | 0.50 | 0.40 |
| C16-40 alkyl stearate | | 1.50 | 1.50 | 1.50 |
| active ingredient | 0.5 | 3.0 | 1.0 | 5.0 |
| Uvinul® A Plus^{™} | 2.00 | 1.50 | 0.75 | 9.00 |
| UvaSorb® k2A ethylhexyl bis-isopentyl benzoxa zolylphenyl melamine | | 2.00 | | 4.00 |
| ethylhexyl methoxycinnamate Uvinul® MC 80 | | 3.00 | | |
| bis-ethylhexyloxyphenol methoxyphenyl triazine | | 1.50 | | 2.00 |
| butyl methoxydi benzoylmethane | | | 2.00 | |
| dinatrium phenyl dibenzimidazole tetrasulfonate | 2.50 | | 0.50 | 2.00 |
| ethyhexyl triazone Uvinul® T 150 | 4.00 | | 3.00 | 4.00 |
| octocrylene | | 4.00 | | |
| diethylhexyl butamido triazone | 1.00 | | | 2.00 |
| phenylbenzimidazol sulfonic acid | 0.50 | | 3.00 | |
| methylene bis-benzotriazolyl tetramethylbutylphenol | 2.00 | | 0.50 | 1.50 |
| ethylhexyl salicylate | | | 3.00 | |
| drometrizol trisiloxane | | | 0.50 | |
| terephthaliden dicamphor sulfonic acid | | 1.50 | | 1.00 |
| diethylhexyl 2,6-naphthalate | | | 7.00 | |
| titanium dioxide-microfine | 1.00 | | 3.00 | |
| zinc oxide- microfine | | | | 0.25 |
| vitamin E acetate | 0.50 | 1.00 | | |
| ascorbyl palmitate | 0.05 | | 0.05 | |
| Buxux chinensis (jojoba) oil | 2.00 | 1.00 | | 1.00 |
| perfume oil, BHT | 0.10 | 0.25 | | 0.35 |
| Ricinus communis (castor) oil | ad 100 | ad 100 | ad 100 | ad 100 |

In a further preferred embodiment, a cosmetic composition comprises a PIT emulsion, which may contain, for example, the following ingredients in % in accordance with the International Nomenclature of Cosmetic Ingredients, INCI:

| **Ingredients (in %)** | **Expl. 1** | **Expl. 2** | **Expl. 3** | **Expl. 4** | **Expl. 5** | **Expl. 6** | **Expl. 7** | **Expl. 8** |
|---|---|---|---|---|---|---|---|---|
| glyceryl monostearate SE | 0.50 | 2.00 | 3.00 | 5.00 | | 0.50 | 4.00 | |
| glyceryl isostearate | | | | | 3.50 | 4.00 | 2.00 | |
| isoceteth-20 | | 0.50 | | | 2.00 | | | |
| ceteareth-12 | | 5.00 | | 1.00 | | | 3.50 | 5.00 |
| ceteareth-20 | | 5.00 | | 1.00 | | | | 3.50 |
| PEG-100 stearate | | | | 2.80 | | 2.30 | 3.30 | |
| cetyl alcohol | 5.20 | | 1.20 | 1.00 | 1.30 | | 0.50 | 0.30 |
| cetyl palmitate | 2.50 | 1.20 | | 1.50 | | 0.50 | | 1.50 |
| cetyl dimethicone copolyol | | | | 0.50 | | 1.00 | | |
| polyglyceryl-2-dioleate | | | | 0.75 | 0.30 | | | |
| active ingredient | 0.1 | 5.0 | 0.01 | 0.5 | 3.0 | 0.25 | 10.0 | 3.0 |
| Uvinul® A Plus^{™} | 2.00 | 1.50 | 0.75 | 1.00 | 2.10 | 4.50 | 5.00 | 2.10 |
| UvaSorb® k2A ethylhexyl bis-isopentylbenzoxazolyl phenyl melamine | | | 4.00 | | | | 1.50 | |
| ethylhexyl methoxycinnamate Uvinul® MC 80 | | | | | 5.00 | 6.00 | 8.00 | 5.00 |
| bis-ethylhexyloxyphenol methoxyphenyl triazine | | 1.50 | | 2.00 | 2.50 | | 2.50 | 2.50 |
| butyl methoxydibenzoylmet hane | | | 2.00 | | 2.00 | 1.50 | | 2.00 |
| dinatrium phenyl dibenzimidazole tetrasulfonate | 2.50 | | 0.50 | 2.00 | | 0.30 | | |
| ethyhexyl triazone Uvinul® T 150 | 4.00 | | 3.00 | 4.00 | | 2.00 | | |
| octocrylene | | 4.00 | | | | | 7.50 | |
| diethylhexyl butamido | 1.00 | | | 2.00 | 1.00 | | 1.00 | 1.00 |
| triazone | | | | | | | | |
| phenylbenzimidazol sulfonic acid | 0.50 | | 3.00 | | | | | |
| methylene bis-benzotriazolyl tetramethylbutylphen ol | 2.00 | | 0.50 | 1.50 | 2.50 | | | 2.50 |
| ethylhexyl salicylate | | | 3.00 | | | | 5.00 | |
| drometrizol trisiloxane | | | 0.50 | | | 1.00 | | |
| terephthalylidene dicamphor sulfonic acid | | 1.50 | | 1.00 | 1.00 | | 0.50 | 1.00 |
| diethylhexyl 2,6-naphthalate | | | 7.00 | | 10.00 | 7.50 | | 8.00 |
| titanium dioxide - microfine | 1.00 | | 3.00 | | 3.50 | | 1.50 | 3.50 |
| zinc oxide - microfine | | | | 0.25 | | 2.00 | | |
| C12-15 alkyl benzoate | 3.50 | | | 6.35 | | | | 0.10 |
| cocoglyceride | | 3.00 | | 3.00 | | | | 1.00 |
| dicapryl ether | 4.50 | | | | | | | |
| dicaprylyl carbonate | | 4.30 | | 3.00 | | | | 7.00 |
| dibutyl adipate | | | | 0.50 | | | | 0.30 |
| phenyl trimethicone | 2.00 | | | 3.50 | | 2.00 | | |
| cyclomethicone | | 3.00 | | | | | | |
| C1-5 alkyl galactomannan | | 0.50 | | | 2.00 | | | |
| hydrogenated coco-glycerides | | | | | 3.00 | 4.00 | | |
| behenoxy dimethicone | | | | | | 1.50 | 2.00 | |
| VP/hexadecene | | | | 1.00 | 1.20 | | | |
| copolymer | | | | | | | | |
| glycerol | 4.00 | 6.00 | 5.00 | | 8.00 | 10.00 | | |
| vitamin E acetate | 0.20 | 0.30 | 0.40 | | 0.30 | | | |
| butyrospermum parkii (shea butter) | | 2.00 | | 3.60 | | 2.00 | | |
| iodopropyl butylcarbamate | 0.12 | | | | 0.20 | | | |
| biosaccaride gum-1 | | | | 0.10 | | | | |
| DMDM hydantoin | 0.10 | | | | 0.12 | | 0.13 | |
| methylparaben | | 0.50 | 0.30 | | 0.35 | | | |
| phenoxyethanol | 0.50 | 0.40 | | 1.00 | | | | |
| ethylhexylglycerol | | 0.30 | | | 1.00 | | 0.35 | |
| ethanol | 2.00 | | 2.00 | | | 5.00 | | |
| perfume oil | 0.20 | | 0.20 | | 0.24 | 0.16 | 0.10 | 0.10 |
| aqua dem. | ad 100 | ad 100 | ad 100 | ad 100 | ad 100 | ad100 | ad 100 | ad 100 |

In a further preferred embodiment, a cosmetic composition comprises a gel creme, which may contain, for example, the following ingredients in % in accordance with the International Nomenclature of Cosmetic Ingredients, INCI:

| **Ingredients (in %)** | **Example 1** | **Example 2** | **Example 3** | **Example 4** |
|---|---|---|---|---|
| acrylates/C10-30 alkylacrylate crosspolymer | 0.40 | 0.35 | 0.40 | 0.35 |
| carbomer | 0.20 | 0.22 | 0.20 | 0.22 |
| Luvigel® EM | 1.50 | 2.50 | 2.80 | 3.50 |
| xanthan gum | 0.10 | 0.13 | 0.10 | 0.13 |
| cetearyl alcohol | 3.00 | 2.50 | 3.00 | 2.50 |
| C12-15 alkylbenzoate | 4.00 | 4.50 | 4.00 | 4.50 |
| caprylic/capric triglyceride | 3.00 | 3.50 | 3.00 | 3.50 |
| titanium dioxide- microfine | 1.00 | | 1.50 | |
| zinc oxide- microfine | | 2.00 | | 0.25 |
| active ingredient | 0.5 | 10.0 | 3.0 | 5.0 |
| dihydroxyacetone | | | 3.00 | 5.00 |
| cyclomethicone | 5.00 | 5.50 | 5.00 | 5.50 |
| dimethicone | 1.00 | 0.60 | 1.00 | 0.60 |
| glycerol | 1.00 | 1.20 | 1.00 | 1.20 |
| sodium hydroxide | q.s. | q.s. | q.s. | q.s. |
| preservative | 0.30 | 0.23 | 0.30 | 0.23 |
| perfume oil | 0.20 | | 0.20 | |
| aqua dem. | ad 100 | ad 100 | ad 100 | ad 100 |
| pH adjusted to 6.0 | | | | |

In a further preferred embodiment, a cosmetic composition comprises a hydrodispersion after sun, which may contain, for example, the following ingredients in % in accordance with the International Nomenclature of Cosmetic Ingredients, INCI:

| **Ingredients (in %)** | **Example 1** | **Example 2** | **Example 3** | **Example 4** | **Example 5** |
|---|---|---|---|---|---|
| ceteaereth-20 | 1.00 | | | 0.50 | |
| cetyl alcohol | | | 1.00 | | |
| Luvigel® EM | | 2.00 | | 2.50 | 2.00 |
| acrylates/C10-30 alkyl acrylate crosspolymer | 0.50 | 0.30 | 0.40 | 0.10 | 0.50 |
| xanthan gum | | 0.30 | 0.15 | | |
| active ingredient | 0.1 | 5.0 | 0.5 | 3.0 | 1.0 |
| C12-15 alkyl benzoate | 2.00 | 2.50 | | | |
| dicapryl ether | | 4.00 | | | |
| butylenglycol dicaprylate/dicaprate | 4.00 | | 2.00 | 6.00 | |
| dicapryl carbonate | | 2.00 | 6.00 | | |
| dimethicone | | 0.50 | 1.00 | | |
| phenyl trimethicone | 2.00 | | 0.50 | | |
| tricontanyl pvp | 0.50 | | 1.00 | | |
| ethylhexylglycerol | | | 1.00 | | 0.80 |
| glycerol | 3.00 | 7.50 | | 7.50 | 8.50 |
| glycine soja (soybean) oil | | | 1.50 | | 1.00 |
| vitamin E acetate | 0.50 | | 0.25 | | 1.00 |
| glucosylrutin | 0.60 | | | 0.25 | |
| ethanol | 15.00 | 10.00 | 8.00 | 12.00 | 9.00 |
| perfume oil | 0.20 | | 0.05 | 0.40 | |
| aqua dem. | ad 100 | ad 100 | ad 100 | ad 100 | ad 100 |

In a further preferred embodiment, a cosmetic composition comprises a W/O emulsion, which may contain, for example, the following ingredients in % in accordance with the International Nomenclature of Cosmetic Ingredients, INCI:

| **Ingredients (in %)** | **Example 1** | **Example 2** | **Example 3** | **Example 4** | **Example 5** |
|---|---|---|---|---|---|
| cetyl dimethicone | | 2.50 | | 4.00 | |
| polyglyceryl-2 dipolyhydroxystearate | 5.00 | | | | 4.50 |
| PEG-30 dipolyhydroxystearate | | | 5.00 | | |
| active ingredient | 5.0 | 10.0 | 0.1 | 0.5 | 1.0 |
| Uvinul® A Plus^{™} | 2.00 | 1.50 | 0.75 | 1.00 | 2.10 |
| titanium dioxid e-microfine | 1.00 | | 3.00 | | 3.50 |
| zinc oxide- microfine | | 0.90 | | 0.25 | |
| minera oil | | 12.00 | 10.00 | | 8.00 |
| C12-15 alkyl benzoate | | | | 9.00 | |
| dicaprylyl ether | 10.00 | | | | 7.00 |
| butylenglycol dicaprylate/dicaprate | | | 2.00 | 8.00 | 4.00 |
| dicaprylyl carbonate | 5.00 | | 6.00 | | |
| dimethicone | | 4.00 | 1.00 | 5.00 | |
| cyclomethicone | 2.00 | 25.00 | | | 2.00 |
| butyrospermum parkii (shea butter) | | | 3.00 | | |
| petrolatum | | 4.50 | | | |
| VP/hexadecene copolymer | 0.50 | | | 0.50 | 1.00 |
| ethylhexylglycerol | | 0.30 | 1.00 | | 0.50 |
| glycerol | 3.00 | 7.50 | | 7.50 | 8.50 |
| glycine soja (soybean) oil | | 1.00 | 1.50 | | 1.00 |
| magnesium sulfate | 1.00 | 0.50 | | 0.50 | |
| magnesium chloride | | | 1.00 | | 0.70 |
| vitamin E acetate | 0.50 | | 0.25 | | 1.00 |
| ascorbyl palmitate | 0.50 | | | 2.00 | |
| biosaccaride gum-1 | | | | 3.50 | 7.00 |
| DMDM hydantoin | | 0.60 | 0.40 | 0.20 | |
| methylparaben | 0.50 | | 0.25 | 0.15 | |
| phenoxyethanol | 0.50 | 0.40 | | 1.00 | |
| ethanol | 3.00 | | 1.50 | | 5.00 |
| perfume oil | 0.20 | | 0.40 | 0.35 | |
| aqua dem. | ad 100 | ad 100 | ad 100 | ad 100 | ad 100 |

In a further preferred embodiment, a cosmetic composition comprises a pickering emulsion, which may contain, for example, the following ingredients in % in accordance with the International Nomenclature of Cosmetic Ingredients, INCI:

| **Ingredients (in %)** | **Example 1** | **Example 2** | **Example 3** | **Example 4** | **Example 5** |
|---|---|---|---|---|---|
| mineral oil | | | 16.00 | 16.00 | |
| octyldodecanol | 9.00 | 9.00 | 5.00 | | |
| caprylic/capric triglyceride | 9.00 | 9.00 | 6.00 | | |
| C12-15 alkyl benzoate | | | | 5.00 | 8.00 |
| butylene glycol dicaprylate/dicaprate | | | | | 8.00 |
| dicaprylyl ether | 9.00 | | | 4.00 | |
| dicaprylyl carbonate | | 9.00 | | | |
| hydroxyoctacosanyl hydroxystearate | 2.00 | 2.00 | 2.20 | 2.50 | 1.50 |
| disteardimonium hectorite | 1.00 | 0.75 | | 0.50 | 0.25 |
| cera microcristallina + paraffnum liquidum | | 0.35 | | | 5.00 |
| hydroxypropyl methylcellulose | | | 0.10 | | 0.05 |
| dimethicone | | | | | 3.00 |
| active ingredient | 1.0 | 0.5 | 0.1 | 3.0 | 5.0 |
| titanium dioxide + alumina + simethicone + aqua | | 3.00 | | | |
| titanium dioxide + trimethoxycaprylylsilane | | 2.00 | 4.00 | 2.00 | 4.00 |
| silica dimethyl silylate | 2.50 | | | 6.00 | 2.50 |
| boron nitride | | | 1.00 | | |
| starch/sodium metaphosphate polymer | 2.00 | | | | |
| tapioca starch | | 0.50 | | | |
| sodium chloride | 5.00 | 7.00 | 8.50 | 3.00 | 4.50 |
| glycerol | | | | 1.00 | |
| vitamin E acetate | 5.00 | 10.00 | 3.00 | 6.00 | 10.00 |
| ascorbyl palmitate | 1.00 | 1.00 | | 1.00 | |
| methylparaben | | 0.60 | | | 0.20 |
| propylparaben | | | | | 0.20 |
| phenoxyethanol | | | 0.20 | | |
| hexamidine diisethionate | | | 0.40 | 0.50 | 0.40 |
| diazolidinyl urea | | | | | 0.08 |
| ethanol | | | 0.23 | 0.20 | |
| perfume oil | 5.00 | | 3.00 | 4.00 | |
| aqua dem. | 0.20 | | 0.30 | 0.10 | |

In a further preferred embodiment, a cosmetic composition comprises a stick, which may contain, for example, the following ingredients in % in accordance with the International Nomenclature of Cosmetic Ingredients, INCI:

| **Ingredients (in %)** | **Example 1** | **Example 2** | **Example 3** | **Example 4** |
|---|---|---|---|---|
| caprylic/capric triglyceride | 12.00 | 10.00 | 6.00 | |
| octyldodecanol | 7.00 | 14.00 | 8.00 | 3.00 |
| butylene glycol dicaprylate/dicaprate | | | | 12.00 |
| pentaerythrityl tetraisostearate | 10.00 | 6.00 | 8.00 | 7.00 |
| polyglyceryl-3 diisostearate | 2.50 | | | |
| bis-diglyceryl polyacyladipate-2 | 9.00 | 8.00 | 10.00 | 8.00 |
| cetearyl alcohol | 8.00 | 11.00 | 9.00 | 7.00 |
| myristyl myristate | 3.50 | 3.00 | 4.00 | 3.00 |
| beeswax | 5.00 | 5.00 | 6.00 | 6.00 |
| copemicia cerifera(carnauba) wax | 1.50 | 2.00 | 2.00 | 1.50 |
| cera alba | 0.50 | 0.50 | 0.50 | 0.40 |
| C16-40 alkyl stearate | | 1.50 | 1.50 | 1.50 |
| active ingredient | 10.0 | 1.0 | 3.0 | 0.1 |
| Uvinul® A Plus^{™} | 2.00 | 1.50 | 0.75 | 9.00 |
| titanium dioxide - microfine | 1.00 | | 3.00 | |
| zinc oxide- microfine | | 1.00 | | 0.25 |
| vitamin E acetate | 0.50 | 1.00 | | |
| ascorbyl palmitate | 0.05 | | 0.05 | |
| Buxux chinensis (jojoba) oil | 2.00 | 1.00 | | 1.00 |
| perfume oil, BHT | 0.10 | 0.25 | | 0.35 |
| Ricinus communis (castor) oil | ad 100 | ad 100 | ad 100 | ad 100 |

In a further preferred embodiment, a cosmetic composition comprises an oil gel, which may contain, for example, the following ingredients in % in accordance with the International Nomenclature of Cosmetic Ingredients, INCI:

| **Ingredients (in %)** | **Example 1** | **Example 2** | **Example 3** | **Example 4** |
|---|---|---|---|---|
| caprylic/capric triglyceride | 12.00 | 10.00 | 6.00 | |
| octyldodecanol | 7.00 | 14.00 | 8.00 | 3.00 |
| butylene glycol dicaprylate/dicaprate | | | | 12.00 |
| pentaerythrityl tetraisostearate | 10.00 | 6.00 | 8.00 | 7.00 |
| polyglyceryl-3 diisostearate | 2.50 | | | |
| bis-diglyceryl polyacyladipate-2 | 9.00 | 8.00 | 10.00 | 8.00 |
| myristyl myristate | 3.50 | 3.00 | 4.00 | 3.00 |
| quatemium-18 bentonite | 5.00 | 5.00 | 6.00 | 6.00 |
| propylene carbonate | 15.00 | 20.00 | 18.00 | 19.50 |
| active ingredient | 1.0 | 0.5 | 3.0 | 5.0 |
| vitamin E acetate | 0.50 | 1.00 | | |
| ascorbyl palmitate | 0.05 | | 0.05 | |
| Buxus chinensis (jojoba) oil | 2.00 | 1.00 | | 1.00 |
| perfume oil, BHT | 0.10 | 0.25 | | 0.35 |
| Ricinus communis (castor) oil | ad 100 | ad 100 | ad 100 | ad 100 |

The present invention also relates to the use of a microorganism according to the invention or of a derivative, mutant or inactive form thereof as described herein above for the preparation of a pharmaceutical composition for suppressing or treating axillar odor.

In another aspect the present disclosure relates to the use of a microorganism according to the invention or of a derivative, mutant or inactive form thereof as described herein above in the context of textiles or textile substrates. Preferably, the present disclosure to the use of a microorganism according to the disclosure or of a derivative, mutant or inactive form thereof as described herein above for the conditioning or impregnation of textiles or textile substrates. More preferably, the microorganism according to the dislcosure or a derivative, mutant or inactive form thereof as described herein above may be applied into or onto textiles or textile substrates according to any suitable methods known to the person skilled in the art or as exemplified herein below. Therefore the present disclosure relates to any of the uses, compositions or methods as described herein above in the ambit of textiles or textile substrates.

Accordingly, the present disclosure relates to a method for the production of textiles and textile substrates for suppressing the release of 3-methyl-2-hexenoic acid by axillary bacteria comprising the steps of formulating a microorganism according to the disclosure or a mutant, derivative or inactive form of this microorganism as described above with textiles and textile substrates. Preferably, said textiles and textile substrates may comprise a cosmetically or pharmaceutical acceptable carrier or excipient as described herein above or comprise one or more of the cosmetic or pharmaceutical compositions as described herein above.

The term "textile and textile substrates for suppressing the release of 3-methyl-2-hexenoic acid by axillary bacteria", as used in accordance with the present disclosure relates to (a) textile composition(s) which comprise(s) at least one microorganism of the present invention or mutant, derivative or inactive form of said microorganism as described above. It is envisaged that the textile compositions of the present disclosure comprise the aforementioned ingredients in any combination. It may, optionally, comprise at least one further ingredient suitable for suppressing the release of 3-methyl-2-hexenoic acid by axillary bacteria (see also Ullmann, Vol. A 26 S. 227 ff, 1995,

According to the present disclosure, textiles and textile substrates are textile fibres, semi-finished and finished textiles and finished products produced therefrom also comprising - apart from textiles for the clothing industry - for example, carpets and other home fabrics and textile formations serving technical purposes. These formations also include unshaped formations such as flocks, linear formations such threads, fibres, yarns, linens, cords, ropes, ply yarns and solid formations such as, for example, felts, woven fabrics, hosiery, knitted fabrics, bonded fibre fabrics and wadding. The textiles can be made, for example, of materials of natural origin, e.g., cotton wool, wool or flax, or synthetic, e.g., polyamide, polyester, modified polyester, polyester blended fabrics, polyamide blended fabrics, polyacrylonitrile, triacetate, acetate, polycarbonate, polypropylene, polyvinyl chloride, polyester microfibres or glass fibre fabrics.

In an embodiment of the present disclosure, the method for the production of textiles and textile substrates for suppressing the release of 3-methyl-2-hexenoic acid by axillary bacteria according to the disclosure may be carried out with any machine or apparatus for the finishing of textiles known to the skilled person, for example standard machines such as foulards. Preferably said foulards are foulard machines with, e.g., vertical infeed, which contain, for example, as essential element two rolls pressed together through which the textile is guided. Above the rolls, an aqueous formulation may be filled in which moistens the textile. Typcically, the pressure quetches the textile and ensures a constant application. In another preferred embodiment, in the foulard machines the textile is, for instance, guided first through an immersion bath and subsequently upwards through two rolls pressed together, e.g. in foulards with vertical textile infeed from below. Machines or apparatuses for the finishing of textiles, especially foulard machines, are described, for example, in Hans-Karl Rouette, "Handbuch der Textilveredlung", Deutscher Fachverlag 2003, p. 618 to 620

In a further embodiment of the present disclosure, the method for the production of textiles and textile substrates for suppressing the release of 3-methyl-2-hexenoic acid by axillary bacteria according to the disclosure can be carried out according to any suitable exhaustion method known to the person skilled in the art, such as, for example, spraying, slop padding, kiss-roll or printing. Prefrably, the method for the production of textiles and textile substrates for suppressing the release of 3-methyl-2-hexenoic acid by axillary bacteria according to the disclosure is carried out according to an exhaustion method with a liquor absorption, for example, in the range from 1 to 50%, preferably from 20 to 40%.

In a further embodiment of the present disclosure, the textile can subsequently be treated thermally by any suitable means known to the person skilled in the art, for example by drying at temperatures in the range of 30 to 100°C or by thermal fixing at temperatures in the range of at least 100, preferably at least 101 °C up to 150°C, preferably up to 135°C. In a preferred embodiment, the treatment may be thermal over a period of 10 seconds up to 30 minutes, preferably 30 seconds up to 10 minutes. In further preferred embodiment of the present disclosure, two thermal treatment steps are carried out at different temperatures, for example, in the first step, drying takes place at temperatures in the range of, e.g., 30 to 100°C over a period of, e.g., 10 seconds to 20 minutes, and then fixing takes place at temperatures in the range of, e.g., 101 to 135°C over a period of, e.g., 30 seconds to 3 minutes.

In a preferred embodiment, the further ingredient comprised in the textile and textile substrates which is suitable for suppressing the release of 3-methyl-2-hexenoic acid by axillary bacteria according to the present invention may be a cyclodextrin as described in DE 40 35 378 or DE 10101294.2, an amylose-containing substance as described in EP-A1-1522626.

Typically, cyclodextrins are cyclic oligosaccharides which are formed by the enzymatic degradation of starch. Preferably, the cyclodextrins to be used as ingredients in the textiles or textile substrates according to the invention are [alpha]-, [beta]- or [gamma]-cyclodextrins which consist, for instance, of six, seven or eight, respectively, [alpha]-1,4 linked glucose units. A characteristic property of the cyclodextrin molecules is their ring structure with largely constant dimensions. Typically, the internal diameter of the rings is about 570 pm for [alpha]-cyclodextrin, about 780 pm for [beta]-cyclodextrin and about 950 pm for [gamma]-cyclodextrin. Due to their structure, cyclodextrins are in the position to be able to incorporate guest molecules. In a preferred embodiment these guest molecules may comprise volatile fragrances as known to the person skilled in the art: Preferably, these fragrances include the fragrances as described herein herein below.

In a further preferred embodiment the present disclosure provides the use of amylose-containing substances for modifying the odour properties of textiles or textile substrates according to the disclosure. Preferably, the amylose content is at least 30% by weight, based on the total weight of the substance. The invention also provides a method of modifying the odour properties of textiles according to the present disclosure which is characterized in that the textile is finished with amylose or an amylose-containing substance, preferably with an amylose content of at least 30% by weight. The term "amylose or amylose-containing substance" means any amylose-containing starches, e.g. native starches, modified starches and starch derivatives, whose amylose content is preferably at least 30% by weight. The starch may be native, e.g. maize starch, wheat starch, potato starch, sorghum starch, rice starch or maranta starch, be obtained by partial digestion of native starch or be chemically modified. Also suitable is pure amylose as it is, e.g. enzymatically obtained amylose, e.g. amylose obtained from sucrose. Also suitable are mixtures of amylose and starch, preferably if the total content of amylose is at least 30% by weight, based on the total weight of the mixture. All data in % by weight which refers to amylose or amylose-containing substances, for mixtures of amylose and starch are always based on the total weight of amylose+starch, unless expressly stated otherwise.

Of particular suitability according to the disclosure are amylose-containing substances, in particular amylose and amylose-containing starches, and amylose/starch mixtures whose amylose content is at least 40% by weight and in particular at least 45% by weight, based on the total weight of the substance. Preferably, the amylose content will not exceed 90% by weight and in particular 80% by weight. Such substances are known to the person skilled in the art and commercially available.

To achieve the odour-modifying effect, the textile according to the disclosure may be finished with the amylose-containing substance generally in any suitable amount, known to the person skilled in the art, preferably of at least 0.5% by weight, more preferably at least 1% by weight and in particular at least 2% by weight, in each case based on the weight of the textile. Preferably, the amylose-containing substance may be used in an amount of not more than 25% by weight, often not more than 20% by weight and in particular not more than 15% by weight, based on the weight of the textile so as not to adversely affect the tactile properties of the textile.

In a further preferred embodiment of the disclosure, to improve the odour properties, the textile material according to the invention may be finished with the amylose-containing substance as it is. However, it is also possible to use the amylose-containing substance together with a fragrance in order to achieve a long-lasting pleasant odour, or scent of the textile. Preferably, the procedure involves treating the textile according to the disclosure with the amylose-containing substance or to treat the textile with the microorganism according to the present invention and the amylose-containing substance at the same time. The textile finished in this way may then be treated with a fragrance. As a result, the amylose-containing substance is charged with the fragrance.

In a further preferred embodiment the textile or textile substrate according to the disclosure which is formulated with a microorganism according to the disclosure or a mutant, derivative or inactive form of this microorganism as described above may be finished with a frangrance.

Preferably, the fragrance as used according to any of the above embodiments may be used in an amount which suffices for the desired scent effect, as known to the person skilled in the art. The upper limit is determined by the maximum absorption capacity of the amylose units of the amylose-containing substance used and will generally not exceed 20% by weight and often 10% by weight, based on the amylose content of the substance. If desired, the fragrance is generally used in an amount of from 0.1 to 10% by weight and in particular 0.5 to 5% by weight.

Suitable fragrances are in principle all volatile organic compounds and mixtures of organic compounds which are known as fragrances. A review of fragrances is given in Ullmann's Encyclopedia of Industrial Chemistry, 5th ed. on CD Rom, Flavours and Fragrances, chapter 2, in particular chapters 2.1 to 2.4. Of particular suitability according to the invention are fragrances of aliphatic and cycloaliphatic nature. These include: aliphatic C4-C12-alcohols, e.g. 3-octanol, cis-3-hexen-1-ol, trans-3-hexen-1-ol, 1-octen-3-ol, 2,6-dimethylheptan-2-ol, 1-octen-3-ol, 9-decen-1-ol, 10-undecen-1-ol, 2-trans-6-cis-nonadien-1-ol, aliphatic C6-C13-aldehydes, e.g. hexanal, octanal, nonanal, decanal, undecanal, 2-methyldecanal, 2-methylundecanal, dodecanal and tridecanal, cis-4-heptenal and 10-undecenal, esters of aliphatic C1-C6-carboxylic acids with aliphatic, optionally monounsaturated C1-C8-alcohols such as ethyl formate, cis-3-hexenyl formate, ethyl acetate, butyl acetate, isoamyl acetate, hexyl acetates, 3,5,5-trimethylhexyl acetate, trans-2-hexenyl acetate, cis-3-hexenyl acetate, ethyl propionate, ethyl butyrates, butyl butyrate, isoamyl butyrate, hexyl butyrate, cis-3-hexenyl isobutyrate, ethyl isovalerate, ethyl 2-methylbutyrate, ethyl hexanoate, 2-propenyl hexanoate, ethyl heptanoate, 2-propenyl heptanoate and ethyl octanoate, acyclic terpene hydrocarbons and hydrocarbon alcohols, such as nerol, geraniol, tetrahydrogeraniol, linalool, tetrahydrolinalool, citronellol, lavandulol, myrcenol, farnesol, nerolidol, the formates, acetates, propionates, butyrates, valerates and isobutyrates of these alcohols, the aldehydes corresponding to the abovementioned alcohols, such as citral, citronellal, hydroxydihydrocitronellal, methoxydihydrocitronellal and the dimethyl- and diethylacetals of these aldehydes, such as diethylcitral, methoxydihydrocitronellal-dimethylacetal, also cyclic terpene hydrocarbons, hydrocarbon alcohols and aldehydes. These may also include scents of natural provenance, such as rose oil, lemon oil, lavender oil and oil of cloves scent.

Thus, the present disclosure also relates to textiles or textile substrates comprising a microorganism according to the invention or a derivative, mutant or inactive form thereof as described herein above. "Comprising" may, e.g., mean associated with or incorporating the microorganism according to the invention or of a derivative, mutant or inactive form thereof as described herein above, in particular, in a form as it results from one of the above-described methods.

It is to be understood that this invention is not limited to the particular methodology, protocols, bacteria, vectors, and reagents etc. described herein as these may vary. It is also to be understood that the terminology used herein is for the purpose of describing particular embodiments only, and is not intended to limit the scope of the present invention which will be limited only by the appended claims. Unless defined otherwise., all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland). Throughout this specification and the claims which follow, unless the context requires otherwise, the word "comprises", and variations such as "comprises" and "comprising", will be understood to imply the inclusion of a stated integer or step or group of integers or steps but not the exclusion of any other integer or step or group of integer or step.

It must be noted that as used herein and in the appended claims, the singular forms "a", "an", and "the", include plural referents unless the context clearly indicates otherwise. Thus, for examples, reference to "a reagent" includes one or more of such different reagents, and reference to "the method" includes reference to equivalent steps and methods known to those of ordinary skill in the art that could be modified or substituted for the methods described herein.

A better understanding of the present invention and of its advantages will be obtained from the following examples, which are offered for illustrative purposes only and are not intended to limit the scope of the present invention in any way.

### Example 1

### Preparation of axillary secretion extracts

Freshly extracted axillary sweat is odorless. Axillary odor develops due to bacterial degradation of apocrine secretion by aerobic skin bacteria. Only when bacteria colonising the axilla contact the odor precursor, the typical axillary sweat odor occurs. To perform an *in vitro* test to detect lactic acid bacteria that are able to suppress odor formation, sterile odorless sweat has to be harvested from the sterile axilla.

The axilla was cleaned with PBS buffer containing 0.1 % of Triton X100. After drying, the axilla was sterilized with 70 % ethanol and a clean tissue. After three hours the axillary secretion was collected by washing and rubbing the axilla with 4 times 5 ml 10 % ethanol. Each washing fraction was collected in a glass flask and the fractions were combined and stored at -20°C. This collection procedure was repeated for several days until 200 ml were collected. This diluted axilla secretion was concentrated in a rotary evaporator (Heidolph) at 90 rpm rotation and 15 mbar pressure. The evaporation was performed at 25 °C and the 200 ml diluted non-odorous sweat was concentrated 100 fold. The concentrated axillary secretion was afterwards centrifuged at 5000 x g for 10 min to remove skin residues and other non soluble contents. The presence of odor precursors was verified by hydrolysation of 100 µl of concentrated secret. This hydrolysation led to the release of the odorous branched chain fatty acid 3M2H. The hydrolysate was reacidified and 3M2H was extracted by CHCl₃, concentrated to 10 µl and detected by GC/MS.

### Example 2

### In vitro generation of axillary odor and quantification

To verify the generally accepted principle of odor generation by hydrolysis of fresh odorless axillary secret, 100 µl of concentrated odorless axillary secret was dissolved in 0.5 ml of 5 M aqueous NaOH and heated to 100°C for 20 min under nitrogen. The reaction mixture was then cooled to room temperature and acidified with 50 µl of 6 M HCl and extracted with 3 x 150 µl CHCl₃. The extract was concentrated to 10 µl under nitrogen and analyzed by GC/MS for the presence of 3M2H. The generation of typical axillary sweat odor was verified by sniffing with the nose. The presence of 3M2H was correlated to the generation of typical axillary odor.

For GC/MS analysis a Hewlett-Packard GC 5980 series II / MSD 5971 system equipped with a split/splitless injector and a FFAP colunm, 30 m x 0.53 mm ID was used. The GC was programmed as follows: 100°C for 2 min, 10°C/min to 220°C and held for 20 min. The mass range employed during these analyses is typically m/z 40-400 which is scanned once/sec. A typical run includes 3600 scans. Identification of 3M2H was by comparison of unknown spectra to commercial standards. In addition the relative chromatographic retention time was used as an identification parameter. 3M2H was quantified relative to control samples and to the standard substance.

### Example 3

### Odor release suppression assay

Lactic acid bacteria have been identified that are able to suppress the release of odorous substances by axillary bacteria. The decrease of odorous substances was measured as a decrease in the release of 3M2H mediated by the typical odor generating axillary skin bacteria *Corynebacterium jeikeium* (DSM 7171) in the presence of a selected lactic acid bacterium.

To identify lactic acid bacteria that are able to suppress the release of odorous substances by axillary bacteria the following *in vitro* assay was performed.

*Corynebacterium jeikeium* (DSM 7171), a typical representative of odor generating axillary bacteria, was aerobically cultivated for 30 h in 20 ml BHI broth at 37°C. The culture was centrifuged for 10 min, at 3000 x g and the cell pellet was washed two times in PBS-buffer, pH 7.0. The cell pellet was resuspended in 20 ml PBS-buffer. Lactic acid bacteria were anaerobically cultivated in 150 µl MRS broth for two days at 37°C.

For the assay 50 µl of washed cells of *Corynebacterium jeikeium* (DSM 7171) were mixed with 100 µl of concentrated, odorless axillary secret. Either 100 µl lactic acid bacteria culture or 100 µl of PBS as control were added to each sample. The samples were incubated aerobically at 37°C for 16 h. Afterwards cells were centrifuged and the supernatant was acidified by 6 M HCl and short chain fatty acids were extracted with 3 x 150 µl CHCl₃. The extract was concentrated to 10 µl under nitrogen and analyzed by GC/MS for the presence of 3M2H.

### Media and buffer:

| | |
|---|---|
| BHI-broth | Difco |
| MRS-broth | Difco |
| PBS buffer | 20 mM phosphate, 150 mM NaCl, pH 7.0 |

Applicant's or agent's
file reference L1326 PCT S3 | International application No.
PCT/EP2006/008923

INDICATIONS RELATING TO DEPOSITED MICROORGANISM
OR OTHER BIOLOGICAL MATERIAL

(PCT Rule 13bis)

A. The indications made below relate to the deposited microorganism or other biological material referred to in the description
on page 18 , line 10 to 19 .

B. IDENTIFICATION OF DEPOSIT | Further deposits are identified on an additional sheet [ ]

Name of depositary institution
DSMZ-Deutsche Sammlung von Mikroorganismen und Zellkulturen GmbH

Address of depositary institution (including postal code and country)
Mascheroder Weg 1b
38124 Braunschweig
DE

Date of deposit | Accession Number
September 12, 2005 (12.09.05) | DSM 17598

C. ADDITIONAL INDICATIONS (leave blank if not applicable) | This information is continued on an additional sheet [ ]

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications e.g., "Accession Number of Deposit")

For receiving Office use only | For International Bureau use only

[ ] This sheet was received with the international application | [ ] This sheet was received by the International Bureau on:

Authorized officer | Authorized officer

Form PCT/RO/134 (July1998; reprint January 2004)

Applicant's or agent's
file reference L1326 PCT S3 | International application No. PCT/EP2006/008923

INDICATIONS RELATING TO DEPOSITED MICROORGANISM
OR OTHER BIOLOGICAL MATERIAL

(PCT Rule 13bis)

A. The indications made below relate to the deposited microorganism or other biological material referred to in the description
on page 18 , line 10 to 19 .

B. IDENTIFICATION OF DEPOSIT | Further deposits are identified on an additional sheet [ ]

Name of depositary institution
DSMZ-Deutsche Sammlung von Mikroorganismen und Zellkulturen GmbH

Address of depositary institution (including postal code and country)
Mascheroder Weg 1b
38124 Braunschweig
DE

Date of deposit | Accession Number
September 12, 2005 (12.09.05) | DSM 17567

C. ADDITIONAL INDICATIONS (leave blank if not applicable) | This information is continued on an additional sheet [ ]

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications e.g., "Accession Number of Deposit")

For receiving Office use only | For International Bureau use only

[ ] This sheet was received with the international application | [ ] This sheet was received by the International Bureau on:

Authorized officer | Authorized officer

Form PCT/RO/134 (July1998; reprint January 2004)

Applicant's or agent's
file reference L1326 PCT S3 | International application No. PCT/EP2006/008923

INDICATIONS RELATING TO DEPOSITED MICROORGANISM
OR OTHER BIOLOGICAL MATERIAL

(PCT Rule 13bis)

A. The indications made below relate to the deposited microorganism or other biological material referred to in the description
on page 18 , line 10 to 19 .

B. IDENTIFICATION OF DEPOSIT | Further deposits are identified on an additional sheet [ ]

Name of depositary institution
DSMZ-Deutsche Sammlung von Mikroorganismen und Zellkulturen GmbH

Address of depositary institution (including postal code and country)
Mascheroder Weg 1b
38124 Braunschweig
DE

Date of deposit | Accession Number
September 12, 2005 (12.09.05) | DSM 17568

C. ADDITIONAL INDICATIONS (leave blank if not applicable) | This information is continued on an additional sheet [ ]

D. DESIGNATED STATES FOR WHICH INDICATIONS ARE MADE (if the indications are not for all designated States)

E. SEPARATE FURNISHING OF INDICATIONS (leave blank if not applicable)
The indications listed below will be submitted to the International Bureau later (specify the general nature of the indications e.g., "Accession Number of Deposit")

For receiving Office use only | For International Bureau use only
[ ] This sheet was received with the international application | [ ] This sheet was received by the International Bureau on:
Authorized officer | Authorized officer

Form PCT/RO/134 (July1998; reprint January 2004)

Applicant's or agent's
file reference L1326 PCT S3 | International application No
PCT/EP2006/008923

INDICATIONS RELATING TO DEPOSITED MICROORGANISM
OR OTHER BIOLOGICAL MATERIAL

(PCT Rule 13bis)

A. The indications made below relate to the deposited microorganism or other biological material referred to in the description
on page 18 , line 10 to 19 .

B. IDENTIFICATION OF DEPOSIT | Further deposits are identified on an additional sheet [ ]

Name of depositary institution
DSMZ-Deutsche Sammlung von Mikroorganismen und Zellkulturen GmbH

Address of depositary institution (including postal code and country)
Mascheroder Weg 1b
38124 Braunschweig
DE

Date of deposit | Accession Number
September 12, 2005 (12.09.05) | DSM 17569

C. ADDITIONAL INDICATIONS (leave blank if not applicable) | This information is continued on an additional sheet [ ]

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications e.g., "Accession Number of Deposit")

For receiving Office use only | For International Bureau use only
[ ] This sheet was received with the international application | [ ] This sheet was received by the International Bureau on:
Authorized officer | Authorized officer

Form PCT/RO/134 (July1998; reprint January 2004)

Applicant's or agent's
file reference L1326 PCT S3 | International application No.
PCT/EP2006/008923

INDICATIONS RELATING TO DEPOSITED MICROORGANISM
OR OTHER BIOLOGICAL MATERIAL

(PCT Rule 13bis)

A. The indications made below relate to the deposited microorganism or other biological material referred to in the description
on page 18 , line 10 to 19 .

B. IDENTIFICATION OF DEPOSIT | Further deposits are identified on an additional sheet [ ]

Name of depositary institution
DSMZ-Deutsche Sammlung von Mikroorganismen und Zellkulturen GmbH

Address of depositary institution (including postal code and country)
Mascheroder Weg 1b
38124 Braunschweig
DE

Date of deposit | Accession Number
September 12, 2005 (12.09.05) | DSM 17570

C. ADDITIONAL INDICATIONS (leave blank if not applicable) | This information is continued on an additional sheet [ ]

D. DESIGNATED STATES FOR WHICH INDICATIONS ARE MADE (if the indications are not for all designated States)

E. SEPARATE FURNISHING OF INDICATIONS (leave blank if not applicable)
The indications listed below will be submitted to the International Bureau later (specify the general nature of the indications e.g., "Accession
Number of Deposit")

For receiving Office use only | For International Bureau use only
[ ] This sheet was received with the international application | [ ] This sheet was received by the International Bureau on:
Authorized officer | Authorized officer

Form PCT/RO/134 (July1998; reprint January 2004)

Applicant's or agent's
file reference L1326 PCT S3 | International application No.
PCT/EP2006/008923

INDICATIONS RELATING TO DEPOSITED MICROORGANISM
OR OTHER BIOLOGICAL MATERIAL

(PCT Rule 13bis)

A. The indications made below relate to the deposited microorganism or other biological material referred to in the description
on page 18 , line 10 to 19 .

B. IDENTIFICATION OF DEPOSIT | Further deposits are identified on an additional sheet ☐

Name of depositary institution
DSMZ-Deutsche Sammlung von Mikroorganismen und Zellkulturen GmbH

Address of depositary institution (including postal code and country)
Mascheroder Weg 1b
38124 Braunschweig
DE

Date of deposit | Accession Number
September 12, 2005 (12.09.05) | DSM 17571

C. ADDITIONAL INDICATIONS (leave blank if not applicable) | This information is continued on an additional sheet ☐

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications e.g., "Accession Number of Deposit")

For receiving Office use only | For International Bureau use only
☐ This sheet was received with the international application | ☐ This sheet was received by the International Bureau on:
Authorized officer | Authorized officer

Form PCT/RO/134 (July1998; reprint January 2004)

## Claims

1. Use of a microorganism which is selected from the group consisting of
Lactobacillus plantarum OB-AG-0002 (DSM 17598),
Lactobacillus crispatus OB-AG-003 (DSM 17567),
Lactobacillus acidophilus II OB-AG-0004 (DSM17568),
Lactobacillus acidophilus II OB-AG-0005 (DSM17569),
Lactobacillus acidophilus III OB-AG-0006 (DSM 17570)
and
Lactobacillus delbrückii delbrückii OB-AG-0007 (DSM 17571)
or a mutant thereof, wherein said mutant retains the ability to suppress the release of 3-methyl-2-hexenoic acid or its odorous derivatives by axillary bacteria
or of an inactive form of said microorganism, which is able to suppress the release of 3-methyl-2-hexenoic acid or its odorous derivatives by axillary bacteria for the preparation of a cosmetic or pharmaceutical composition for suppressing axillar odor.

2. Use of an inactive form of said microorganism according to claim 1 which is thermally inactivated or lyophilized.

## Patentansprüche

1. Verwendung eines Mikroorganismus, der ausgewählt ist aus der Gruppe bestehend aus
Lactobacillus plantarum OB-AG-0002 (DSM 17598),
Lactobacillus crispatus OB-AG-003 (DSM 17567),
Lactobacillus acidopholus II OB-AG-0004 (DSM 17568),
Lactobacillus acidopholus II OB-AG-0005 (DSM 17569),
Lactobacillus acidopholus III OB-AG-0006 (DSM 17570),
und
Lactobacillus delbrückii delbrückii OB-AG-0007 (DSM 17571),
oder einer Mutante davon, wobei die Mutante die Fähigkeit beibehält, die Freisetzung von 3-Methyl-2-hexen-Säure oder deren Geruchsderivate durch axilläre Bakterien unterdrücken zu können,
oder einer inaktiven Form des Mikroorganismus, die in der Lage ist, die Freisetzung von 3-Methyl-2-hexen-Säure oder deren Geruchsderivate durch axilläre Bakterien zu unterdrücken,
für die Herstellung einer kosmetischen oder pharmazeutischen Zusammensetzung zum Unterdrücken von axillärem Geruch.

2. Verwendung einer inaktiven Form des Mikroorganismus nach Anspruch 1, die thermisch inaktiviert oder lyophilisiert ist.

## Revendications

1. Utilisation d'un microorganisme, qui est sélectionné à partir du groupe consistant en
Lactobacillus plantarum OB-AG-0002 (DSM 17598),
Lactobacillus crispatus OB-AG-003 (DSM 17567),
Lactobacillus acidopholus II OB-AG-0004 (DSM 17568),
Lactobacillus acidopholus II OB-AG-0005 (DSM 17569),
Lactobacillus acidopholus III OB-AG-0006 (DSM 17570),
et
Lactobacillus delbrückii delbrückii OB-AG-0007 (DSM 17571),
ou un mutant de ceux-ci, le mutant gardant la capacité de pouvoir réprimer la libération d'acide 3-méthyl-2-hexénoïque ou de ses dérivés ayant une odeur désagréable par des bactéries axillaires,
ou une forme inactive de ce microorganisme, qui est capable de réprimer la libération d'acide 3-méthyl-2-hexénoïque ou de ses dérivés ayant une odeur désagréable par des bactéries axillaires,
pour la fabrication d'une composition cosmétique ou pharmaceutique pour réprimer l'odeur axillaire.

2. Utilisation d'une forme inactive de ce microorganisme selon la revendication 1, qui est inactivée thermiquement ou lyophilisée.
